# EUROPEAN PATENT APPLICATION

(11) **EP 0 271 287 A2**
(43) Date of publication of application: **15.06.1988**
(21) Application number: 87310645.4
(22) Date of filing: 03.12.1987
(51) Int. Cl.: C07D 215/14, C07D 215/12, C07D 215/22, C07D 215/18, C07D 215/36, C07D 215/38, C07D 215/48, C07D 401/12, C07D 401/10, A61K 31/47

(54) **Quinoline dioic acids and amides**

(30) Priority: 11.12.1986 US 940709
(71) Applicant: MERCK FROSST CANADA INC., Kirkland Quebec H9H 3L1 (CA)
(72) Inventor: Young, Robert N., Quebec H9X 3L2 (CA); Zamboni, Robert, Longueuil Quebec J4L 1E1 (CA); Williams, Haydn W. R., Dollard des Ormeaux Quebec H9G 1V4 (CA); Belley, Michel L., Apt. 911 St. Laurent H9N 1P1 (CA)
(74) Representative: Cole, William Gwyn

(57) **Abstract**

Compounds having the formula 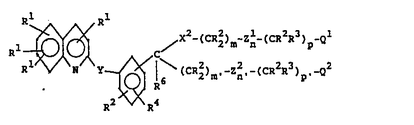 are antagonists of leukotrienes and inhibitors of their biosynthesis. These compounds are useful as anti-asthmatic, anti-allergic, anti-inflammatory, and cytoprotective agents.

## Description

### BACKGROUND OF THE INVENTION

This invention is directed to compounds which act as antagonists of the leukotrienes and inhibitors of their biosynthesis.

The leukotrienes and their biological activities, especially their roles in various disease states and conditions have been described. For example, see EP 140,684 (May 8, 1985), which is incorporated herein by reference. Several classes of compounds exhibit ability to antagonize the action of leukotrienes in mammals, especially humans. See for example: United Kingdom Patent Specification Nos. 2,058,785 and 2,094,301; and European Patent Application Nos. 56,172, 61,800 and 68,739.

EP 110,405 (June 13, 1984) describes anti-inflammatory and antiallergic substituted benzenes which are disclosed to be leukotriene inhibitors, i.e., inhibitors of the 5-lipoxygenase pathway.

### SUMMARY OF THE INVENTION

The present invention relates to compounds having activity as leukotriene and SRS-A antagonists or inhibitors, to methods for their preparation, to intermediates useful in their preparation and to methods and pharmaceutical formulations for using these compounds in mammals (especially humans).

Because of their activity as leukotriene antagonists or inhibitors, the compounds of the present invention are useful as anti-asthmatic, anti-allergic, and anti-inflammatory agents and are useful in treating allergic rhinitis and chronic bronchitis and for amelioration of skin diseases like psoriasis and atopic eczema. These compounds are also useful to antagonize or inhibit the pathologic actions of leukotrienes on the cardiovascular and vascular systems for example, actions such as result in angina. The compounds of the present invention are useful in the treatment of inflammatory and allergic diseases of the eye, including allergic conjunctivitis. The compounds are also useful as cytoprotective agents.

Thus, the compounds of the present invention may also be used to treat or prevent mammalian (especially, human) disease states such as erosive gastritis; erosive esophagitis; inflammatory bowel disease; ethanol-induced hemorrhagic erosions; hepatic ischemic; noxious agent induced damage or necrosis of hepatic, pancreatic, renal, or myocardial tissue; liver parenchymal damage caused by hepatoxic agents such as CC1₄ and D-galactosamine; ischemic renal failure; disease-induced hepatic damage; bile salt induced pancreatic or gastric damage; trauma- or stress-induced cell damage; glycerol-induced renal failure; and migraine

### DETAILED DESCRIPTION

The compounds of this invention are best realized by Formula I: 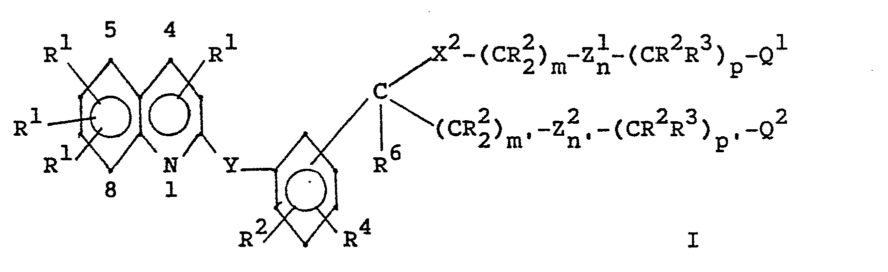 wherein:
R¹ is H, halogen, Ci-C₈ alkyl, C₂-C₈ alkenyl, C₂-C₈ alkynyl, -CF₃, -OR², -SR², -S(O)R², -S(0)_{2R}², -NR²R², -CHO, -COOR², -(C= O)R2, -C(OH)R²R², -CN< -N0₂, substituted or unsubstituted phenyl, substituted or unsubstituted benzyl, or substituted or unsubstituted phenethyl;
R² is H, C₁-C₈ alkyl, C₂-C₈ alkenyl, C₂-Cₐ alkynyl, -CF₃, substituted or unsubstituted phenyl, substituted or unsubstituted benzyl, or substituted or unsubstituted phenethyl;
R³ is H, halogen, -N0₂, -CN, -OR², -SR², NR²R², or C₁-C₈ alkyl;
CR²R³ may be the radical of a naturally occurring amino acid;
R⁴ is H, halogen, -N0₂, -CN, -OR², -SR², NR²R², C₁-C₈ alkyl, or -(C=O)R²;
R⁵ is 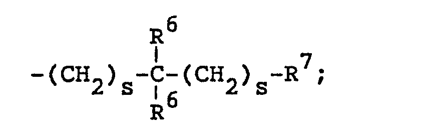
R⁶ is H or C₁-C₄ alkyl;
R⁷ is A) a monocyclic or bicyclic heterocyclic radical containing from 3 to 12 nuclear carbon atoms and 1 or 2 nuclear heteroatoms selected from N, S or 0 and with each ring in the heterocyclic radical being formed of 5 or 6 atoms, or
B) the radical W-R⁸;
R⁸ contains up to 21 carbon atoms and is (1) a hydrocarbon radical or (2) an acyl radical of an organic acyclic or monocyclic carboxylic acid containing not more than 1 heteroatom in the ring;
R⁹ is -OR¹⁰, -SR¹⁰, or NR¹⁰R¹⁰;
R¹⁰ is H, C₁-C₆ alkyl, -(C=O)R¹¹, unsubstituted phenyl, unsubstituted benzyl, or two R¹⁰ groups joined to the same N may form a ring of 5 or 6 members containing up to two heteratoms chosen from 0, S or N;
R¹¹ is H, C₁-C₈ alkyl, C₂-C₈ alkenyl, C₂-C₈ alkynyl, -CF₃, or unsubstituted phenyl, benzyl, or phenethyl;
R12 is R² or halogen;
m and m' are independently 0-8;
n and n' are independently 0 or 1;
p and p' are independently 0-8;
m + n + p is 1-10;
m' + n' + p' is 0-10;
s is 0-3;
Q¹ and Q² are independently -COOR², tetrazole, -COOR⁵, -CONHS(O)₂R¹¹. -CN, -CONR¹⁰R¹⁰, -CHO, -CH₂0H, -COCH₂0H, -NHS(O)₂R¹¹; or if Q¹ or Q² is COOH and R³ is -OH, -SH, or -NHR² then Q¹ or Q² and R³ and the carbons through which they are attached may form a heterocyclic ring with loss of water;
W is 0, S, or NH;
X¹ is 0, S, -S(O)-, -S(O)₂-, -NR², or -CR²R²⁻;
X² is CR²R², 0, S, S(O), or S(0)₂;
Y is -CR²=CR²-, -C=C-, -CR²R²-X¹-, -X¹-CR²R²-, -CR²R²-X¹-CR²R²-, 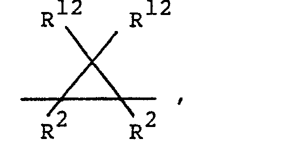 C=O, 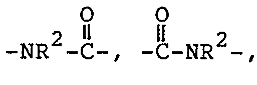 O, S, or -_{NR}²_{;}
Z¹ and Z² are independently -CONR²⁻;
and the pharmaceutically acceptable salts thereof.

Alkyl, alkenyl, and alkynyl are intended to include linear, branched, and cyclic structures.

Alkyl groups include methyl, ethyl, propyl, isopropyl, butyl sec- and tert-butyl, pentyl, hexyl, heptyl, cyclopropyl, cyclobutyl, cyclopentyl, cyclohexyl, cycloheptyl, norbornyl and the like.

Alkenyl groups include vinyl, allyl, isopropenyl, pentenyl, hexenyl, heptenyl, cyclopropenyl, cyclobutenyl, cyclopentenyl, cyclohexenyl, cycloheptenyl, 1-propenyl, 2-butenyl, 2-methyl-2-butenyl, norborneyl, and the like.

Substituted phenyl, benzyl, and phenethyl include 1 or 2 substituents on the benzene ring selected from C₁-C₆ alkyl, R⁹, N0₂, SCF₃, halogen, -COR⁶, -COR⁹, CN, and CF₃.

Halogen includes F, Cl, Br and I.

The prodrug esters of Q (i.e., when Q = -COOR^{S}) are intended to include the esters such as are described by Saari et al., J. Med Chem., 21, No. 8, 746-753 (1978).

When Q and R³ and the carbons through which they are attached form a ring, the rings thus formed include lactones, lactams, and thiolactones.

It is intended that the definitions of any substituent (e.g., R¹, R², m, Q, X, etc.) in a particular molecule be independent of its definitions elsewhere in the molecule. Thus, -NR²R² represents -NHH, -NHCH₃, -NHC₆H₅, etc.

The heterocycles formed when two R¹⁰ groups join through N include pyrrolidine, piperidine, morpholine, thiamorpholine, piperazine, and n-methylpiperazine.

The naturally occurring amino acids, the radicals of which may be CR²R³, include alanine, asparganine, aspartic acid, arginine, cysteine, glutamic acid, glutamine, glycine, histidine, isoleucine, leucine, lysine, methionine, phenylalanine, proline, serine, threonine, tryptophan, tyrosine and valine.

Examples of Z-CR²R³⁻Q containing a naturally occurring amino acid radical include: 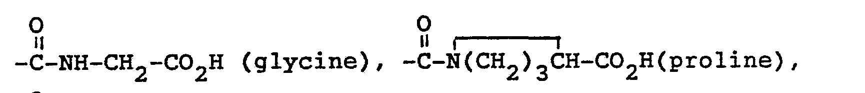 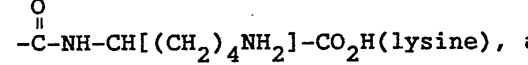 and

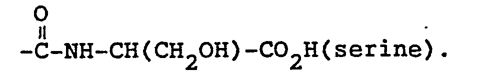

Some of the compounds described herein contain one or more centers of asymmetry and may thus give rise to diastereoisomers and optical isomers. The present invention is meant to comprehend such possible diastereoisomers as well as their racemic and resolved, optically active forms.

Some of the compounds described herein contain olefinic double bonds, and unless specified otherwise, are meant to include both E and Z geometric isomers.

Preferred compounds of Formula I are best represented by Formula la: 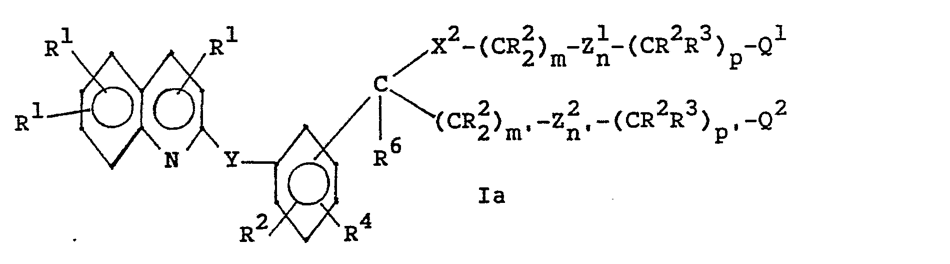 wherein:
Y is -CR²=_{CR}2-, -C=C-, CR 0, or -CR²₂ S-;
the other substituents are as defined for Formula I; and the pharmaceutically acceptable salts thereof.

More-preferred compounds of Formula I are best represented by Formula Ib: 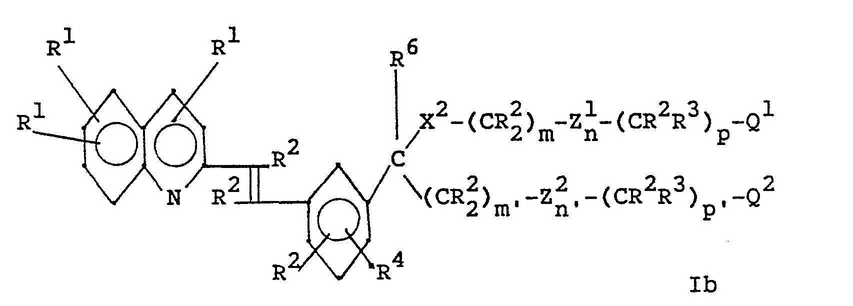 wherein:
R¹ is H, halogen, G₁-C₃alkyl, -CF₃, or SCF₃;
R² is H, C₁-C₃ alkyl, C₂-C₃ alkenyl, or -CF₃;
the other substituents are as defined for Formula I; and the pharmaceutically acceptable salts thereof.

Other more-preferred compounds of Formula I are best represented by Formula Ic: 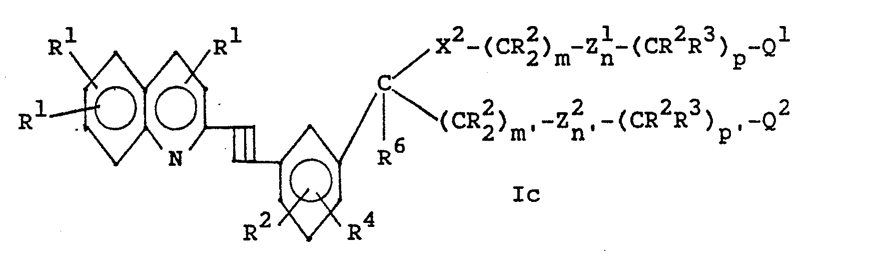 wherein:
R¹ is H, halogen, C₁-C₃ alkyl, -CF₃, or SCF₃;
R² is H, C₁-C₃ alkyl, C₂-C₃ alkenyl, or -CF₃;
the other substituents are as defined for Formula I; and the pharmaceutically acceptable salts thereof.

Other more-preferred compounds within the scope of this invention are best represented by Formula Id: 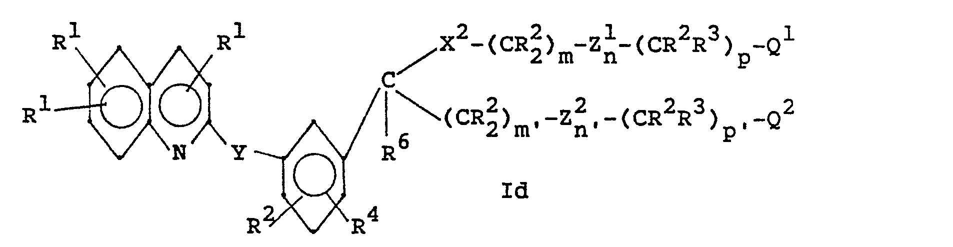 wherein:
R¹ is H, halogen, C₁-C₃ alkyl, CF₃ or SCF₃;
R² is H, C₁-C₃alkyl, C₂-C₃alkenyl, or CF₃;
Y is -CR 0- or -CR S-;
the other substituents are as defined for Formula I; and the pharmaceutically acceptable salts thereof.

A preferred embodiment of compounds of formulae Ib, Ic, or Id is that wherein Q¹ or Q² is COOR², tetrazole, COOR⁵, CONR¹⁰R¹⁰ or COCH₂0H, with the remaining substituent being defined as for Ib, Ic or ld, respectively.

The compounds of Formula I are active as antagonists of SRS-A and especially of leukotriene D₄. These compounds also have modest inhibitory activity on leukotriene biosynthesis but are primarily of therapeutic interest as antagonists. The activity of the compounds of Formula I can be detected and evaluated by methods known in the art. See for example, Kadin, U.S. Patent No. 4,296,129.

The ability of the compounds of Formula I to antagonize the effects of the leukotrienes and to inhibit the leukotrienes makes them useful for inhibiting the symptoms induced by the leukotrienes in a human subject. The compounds are valuable therefore in the prevention and treatment of such disease states in which the leukotrienes are the causative factor, e.g. skin disorders, allergic rhinitis, and obstructive airway diseases. The compounds are particularly valuable in the prevention and treatment of allergic bronchial asthma. They are also effective in the treatment of inflammatory diseases of the eye. It will be understood that in this paragraph and in the discussion of methods of treatment which follows, references to the compounds of Formula I are meant to include the pharmaceutically acceptable salts and lactone, lactam or thiolactam forms.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are; (A) an ethanol-induced lesion assay and (B) an indomethacin-induced ulcer assay and are described in EP 140,684.

The magnitude of a prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range for anti-asthmatic, anti-allergic or anti-inflammatory use and generally, uses other than cytoprotection, lie within the range of from about 0.001 mg to about 100 mg per kg body weight of a mammal, preferably 0.01 mg to about 10 mg per kg, and most preferably 0.1 to 1 mg per kg, in single or divided doses. On the other hand, it may be necessary to use dosages outside these limits in some cases.

The exact amount of a compound of the Formula I to be used as a cytoprotective agent will depend on, inter alia, whether it is being administered to heal damaged cells or to avoid future damage, on the nature of the damaged cells (e.g., gastrointestinal ulcerations vs. nephrotic necrosis), and on the nature of the causative agent. An example of the use of a compound of the Formula I in avoiding future damage would be co-administration of a compound of the Formula I with a non-steroidal anti-inflammatory drug that might otherwise cause such damage (for example, indomethacin). For such use, the compound of Formula I is administered from 30 minutes prior up to 30 minutes after administration of the NSAID. Preferably it is administered prior to or simultaneously with the NSAID, (for example, in a combination dosage form). The effective daily dosage level for compounds of Formula inducing cytoprotection in mammals, especially humans, will generally range from about 0.1 mg/kg to about 100 mg/kg, preferably from about 1 mg/kg to about 100 mg/kg. The dosage may be administered in single or divided individual doses.

Any suitable route of administration may be employed for providing a mammal, especially a human with an effective dosage of a leukotriene antagonist. For example, oral, rectal, topical, parenteral, ocular, nasal, buccal, and the like may be employed. Dosage forms include tablets, troches, dispersions, suspensions, solutions, capsules, creams, ointments, aerosols, and the like.

The pharmaceutical compositions of the present invention comprise a compound of Formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

Salts derived from inorganic bases include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

When the compound of the present invention is basic, salts may be prepared from pharmaceutically acceptable non-toxic acids, including inorganic and organic acids. Such acids include acetic benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric acid, p-toluenesulfonic and the like. Particularly preferred are hydrobromic, hydrochloric, phosphoric and sulfuric acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

For use where a composition for intravenous administration is employed, a suitable dosage range for anti-asthmatic, anti-inflammatory or anti-allergic use is from about 0.001 mg to about 10 mg (preferably from about 0.01 mg to about 1 mg) of a compound of Formula I per kg of body weight per day and for cytoprotective use from about 0.1 mg to about 100 mg (preferably from about 1 mg to about 100 mg and more preferably from about 1 mg to about 10 mg) of a compound of Formula I per kg of body weight per day.

In the case where an oral composition is employed, a suitable dosage range for anti-asthmatic, anti-inflammatory or anti-allergic use is, e.g. from about 0.01 mg to about 100 mg of a compound of Formula I per kg of body weight per day, preferably from about 0.1 mg to about 10 mg per kg and for cytoprotective use from about 0.1 mg to about 100 mg (preferably from about 1 mg to about 100 mg and more preferably from about 10 mg to about 100 mg) of a compound of Formula I per kg of body weight per day.

For administration by inhalation, the compounds of the present invention are conveniently delivered in the form of an aerosol spray presentation from pressurized packs or a nebuliser, or as a powder which may be formulated as a cartridge from which the powder composition may be inhaled with the aid of a suitable device. The preferred delivery system for inhalation is a metered dose inhalation (MDI) aerosol, which may be formulated as a suspension or solution in fluorocarbon propellants.

Suitable topical formulations of Compound I include transdermal devices, aerosols, creams, ointments, lotions, dusting powders, and the like.

For the treatment of diseases of the eye, ophthalmic preparations for ocular administration comprising 0.001-1⁰/o by weight solutions or suspensions of the compounds of Formula I in an acceptable ophthalmic formulation may be used.

In practical use, the compounds of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form, in which case solid pharmaceutical carriers are obviously employed. If desired, tablets may be coated by standard aqueous or nonaqueous techniques.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719, the disclosures of which are hereby incorporated herein by reference.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 2.5 mg to about 500 mg of the active ingredient and each cachet or capsule contains from about 2.5 to about 500 mg of the active ingredient. The following are examples of representative pharmaceutical dosage forms for the compounds of Formula I:

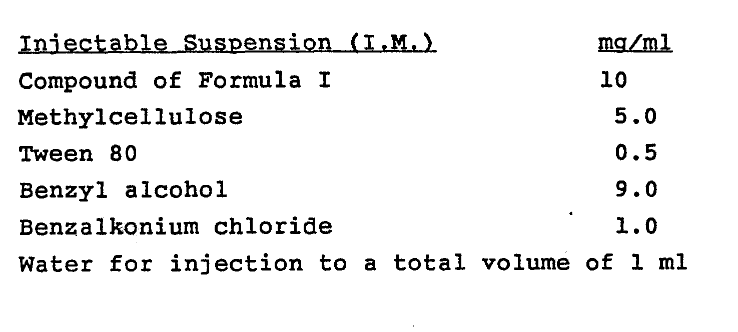

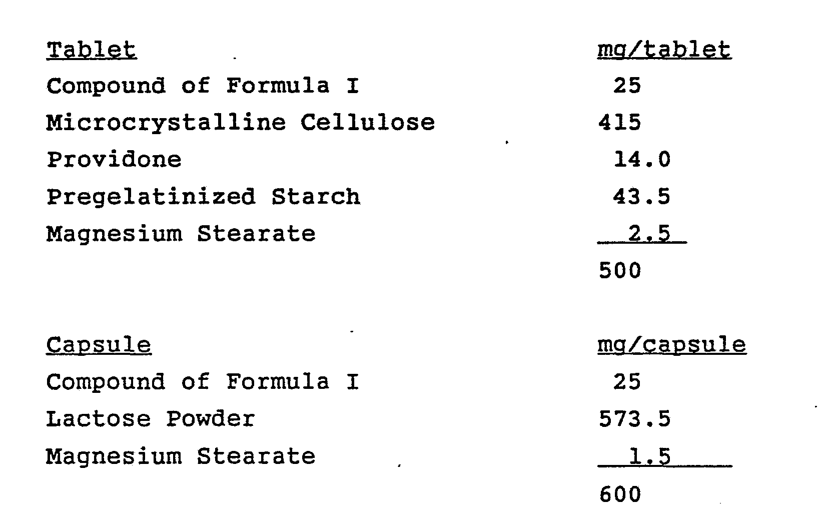

In addition to the compounds of Formula I, the pharmaceutical compositions of the present invention can also contain other active ingredients, such as cyclooxygenase inhibitors, non-steroidal anti-inflammatory drugs (NSAIDs), peripheral analgesic agents such as zomepirac, diflunisal and the like. The weight ratio of the compound of the Formula I to the second active ingredient may be varied and will depend upon the effective dose of each ingredient. Generally, an effective dose of each will be used. Thus, for example, when a compound of the Formula I is combined with an NSAID the weight ratio of the compound of the Formula I to the NSAID will generally range from about 1000:1 to about 1:1000. Combinations of a compound of the Formula I and other active ingredients will generally also be within the aforementioned range, but in each case, an effective dose of each active ingredient should be used.

NSAIDs can be characterized into five groups: (1) the propionic acid derivatives;
(2) the acetic acid derivatives;
(3) the fenamic acid derivatives;
(4) the biphenylcarboxylic acid derivatives; and
(5) the oxicams or a pharmaceutically acceptable salt thereof.

NSAIDs which are within the scope of this invention are those disclosed in EP 140,684. Pharmaceutical compositions comprising the Formula I compounds may also contain inhibitors of the biosynthesis of the leukotrienes such as are disclosed in EP 138,481 (April 24, 1985), EP 115,394 (August 8, 1984), EP 136,893 (April 10, 1985), and EP 140,709 (May 5, 1985), which are hereby incorporated herein by reference.

The compounds of the Formula I may also be used in combination with leukotriene antagonists such as those disclosed in EP 106,565 (April 25, 1984) and EP 104,885 (April 4, 1984) which are hereby incorporated herein by reference and others known in the art such as those disclosed in European Patent Application Nos. 56,172 (July 21,1982) and 61,800 (October 6,1982); and in U.K. Patent Specification No. 2,058,785, which are hereby incorporated herein by reference.

Pharmaceutical compositions comprising the Formula I compounds may also contain as the second active ingredient prostaglandin antagonists such as those disclosed in European Patent Applications 11,067 (May 28, 1980), 166,591 (Jan. 2,1986) or thromboxane antagonists such as those disclosed in U.S. 4,237,160. They may also contain histidine decarboxylase inhibitors such as @-fiuoromethyihistidine, described in U.S. 4,325,961. The compounds of the Formula I may also be advantageously combined with an Hi or H₂-receptor antagonist, such as for instance benadryl, dramamine, histadyl, phenergan, terfenadine, acetamazole, cimetidine, ranitidine, famotidine, aminothiadiazoles disclosed in EP 40,696 (December 2, 1981) and like compounds, such as those disclosed in U.S. Patent Nos. 4,283,408; 4,362,736; and 4,394,508. The pharmaceutical compositions may also contain a K⁺ /H⁺ ATPase inhibitor such as omeprazole, disclosed in U.S. Pat. 4,255,431, and the like. Another useful pharmaceutical composition comprises the Formula I compounds in combination with serotonin antagonists such as methysergide, the serotonin antagonists disclosed in Nature, vol. 316, pages 126-131, 1985, and the like. Each of the references referred to in this paragraph is hereby incorporated herein by reference.

Compounds of the present invention can be prepared according to the following methods.

In these schema, the 2-substituted quinoline radical 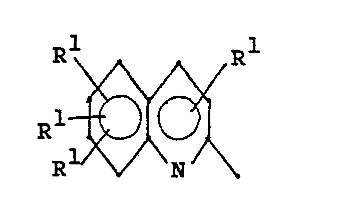 is represented by "2SQ-".

### METHOD A

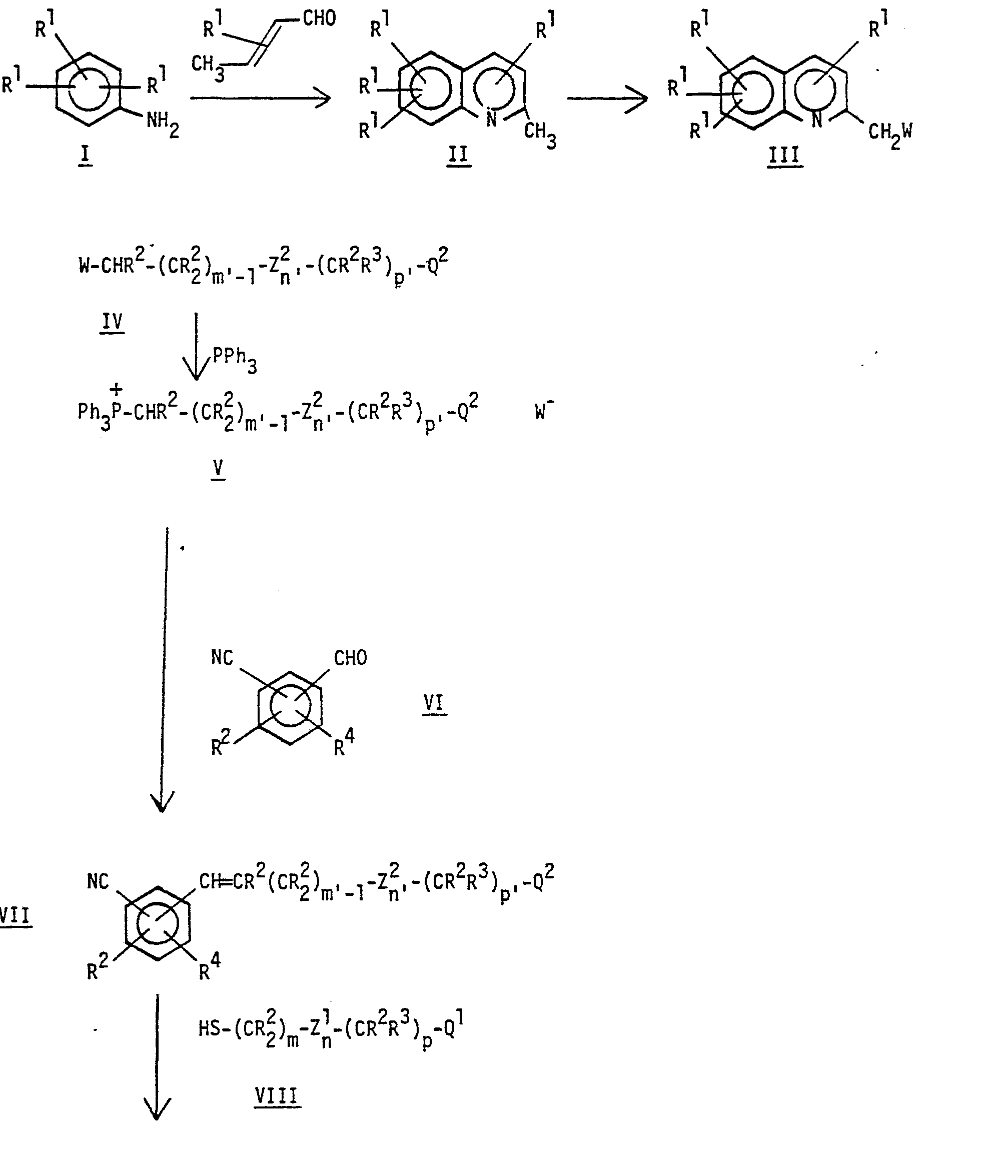

METHOD A (cont'd.)

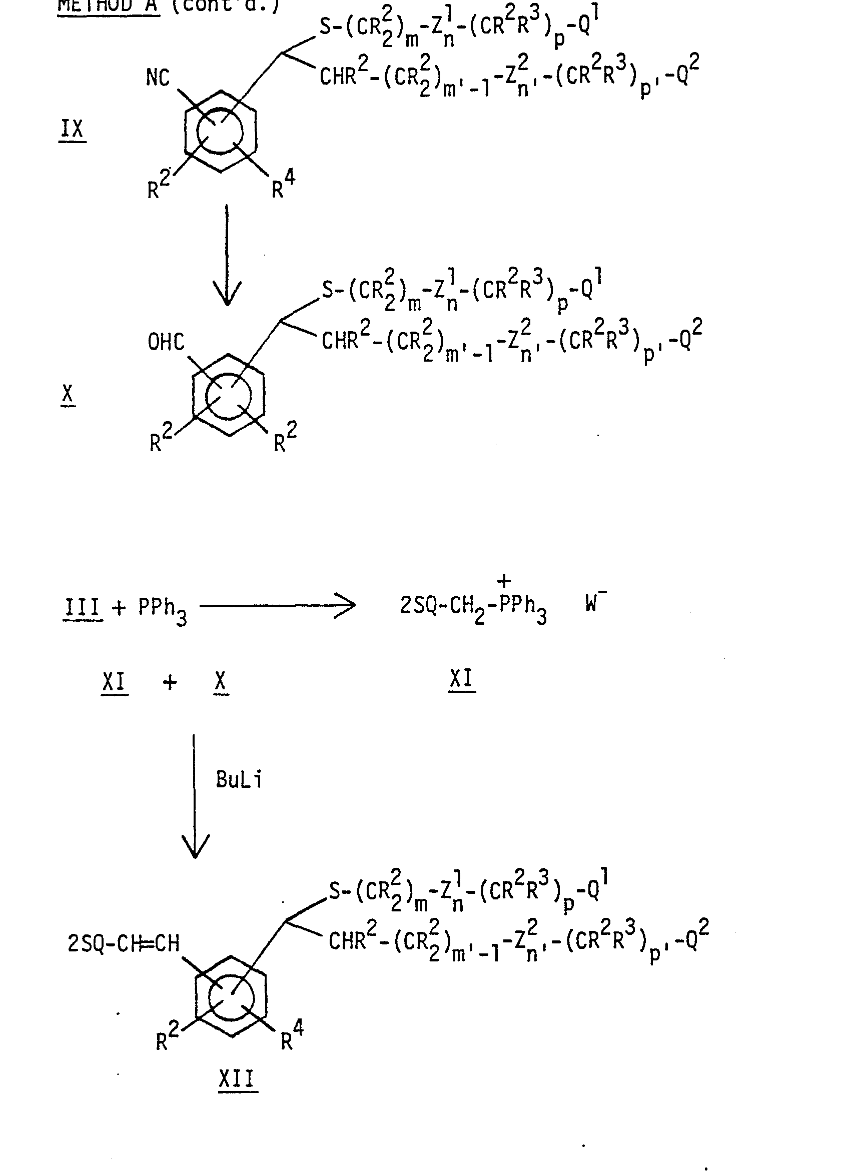

### METHOD B

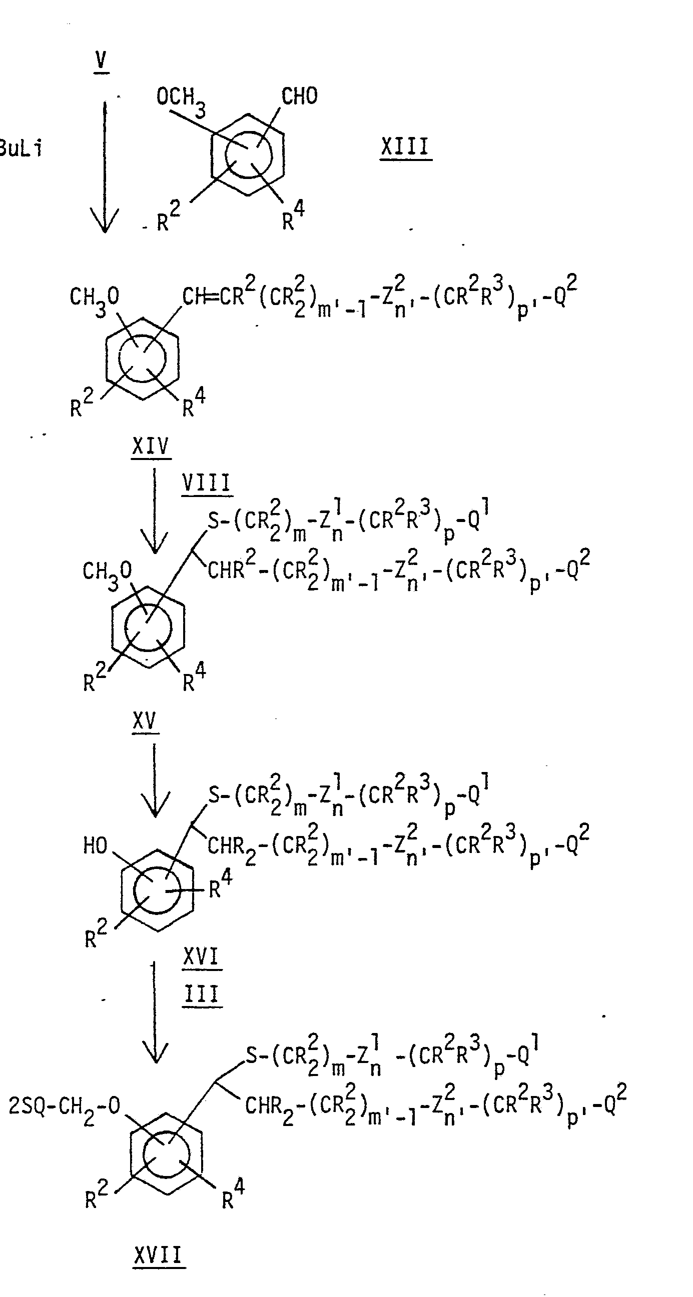

### METHOD C

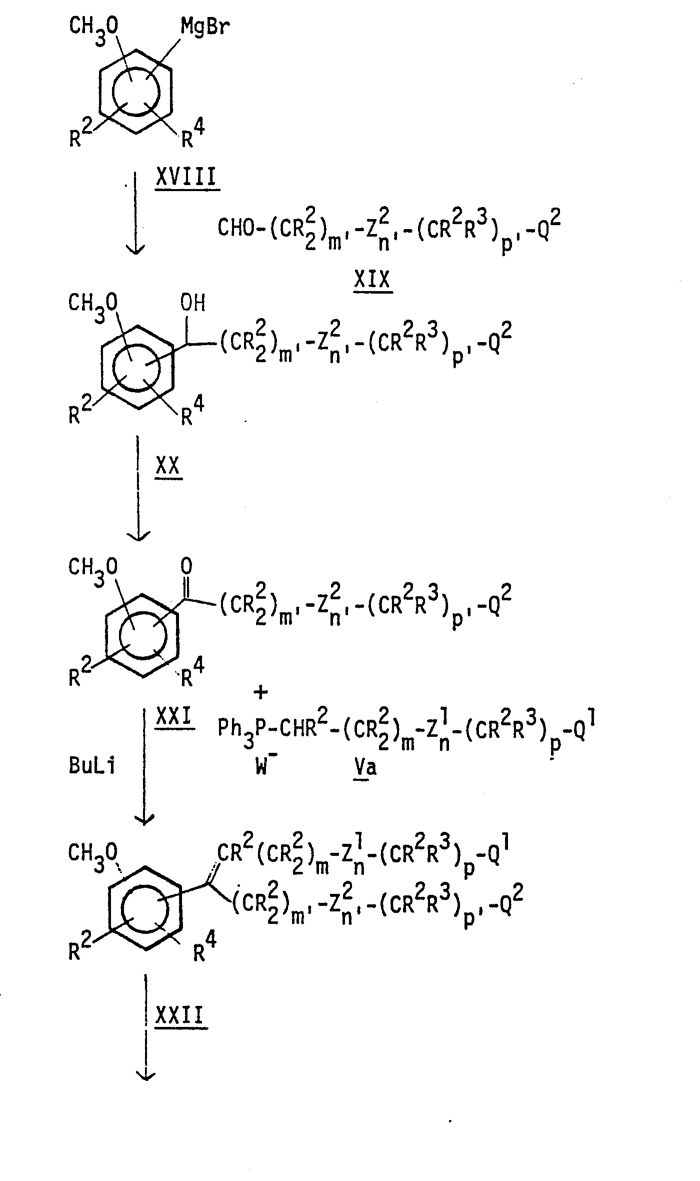

METHOD C (cont'd.)

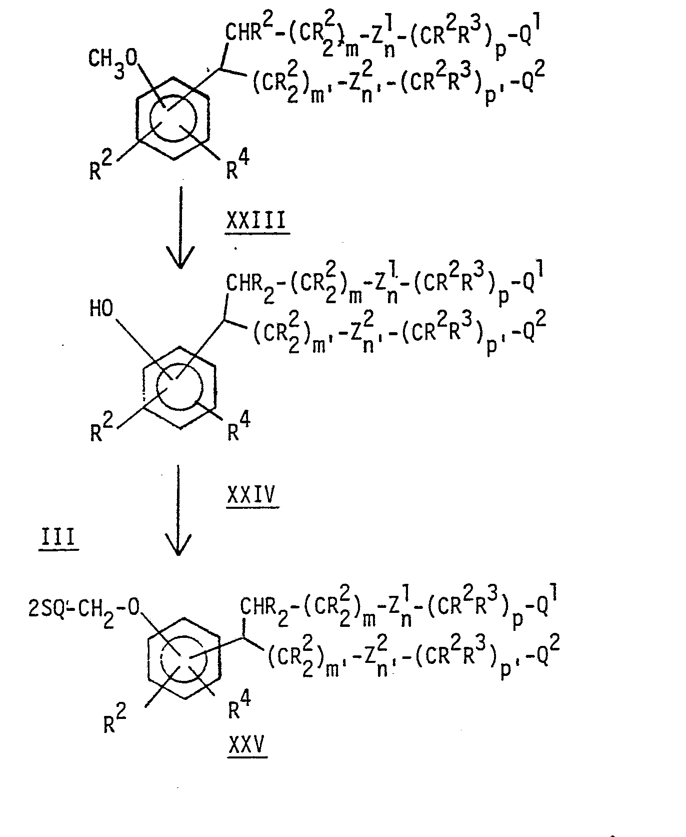

### METHOD D

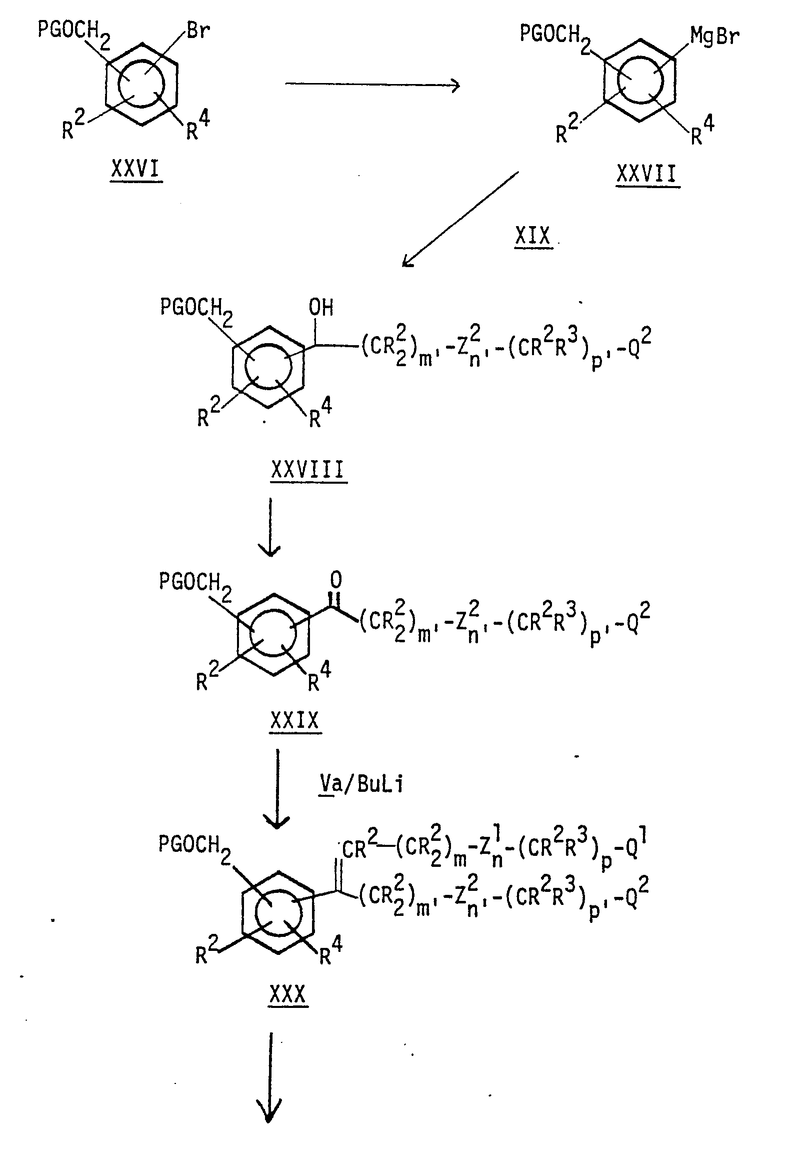

METHOD D (cont'd.)

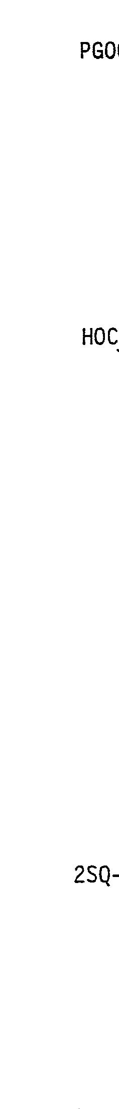

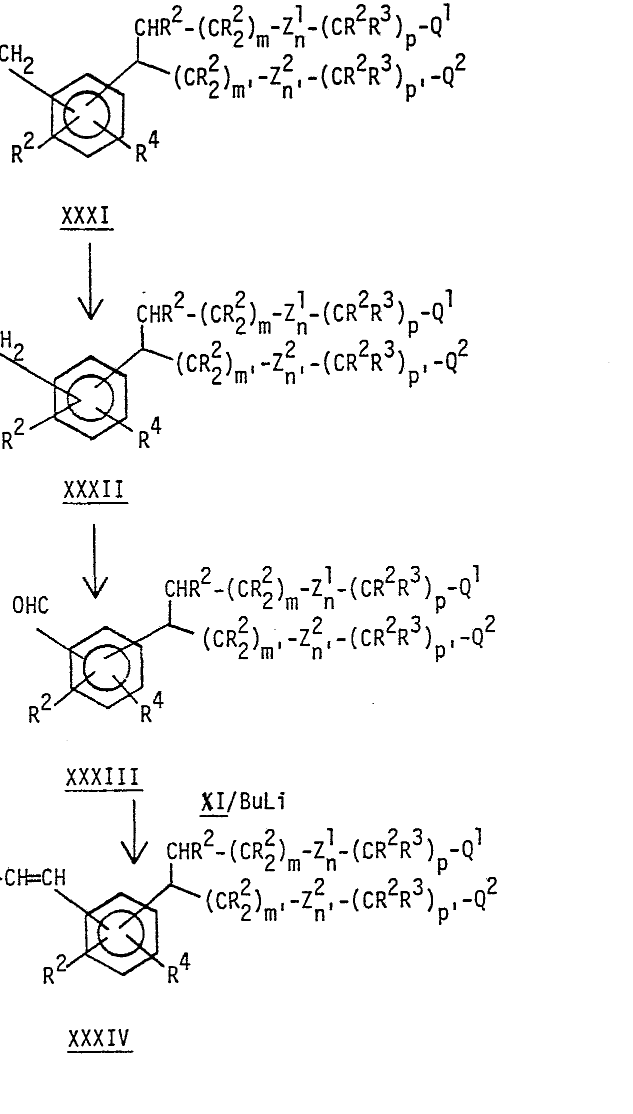

### METHOD A

Referring to Method A, an aniline derivative of Formula I is reacted by heating with a crotonaldehyde and a strong mineral acid such as aqueous hydrochloric acid to provide the substituted quinaldine derivative of structure II. The derivative is treated with a reagent such as NCS, NBS or NIS in the presence of light with a suitable catalyst such as dibenzoyl peroxide with heat to afford derivative of structure III.

A derivative of structure IV which contains a suitable leaving group W like Cl, Br or I is then reacted with triphenyl phosphine in a suitable solvent such as CH₃CN to give phosphonium salt V. The salt V is treated with a strong base such as BuLi or the potassium salt of hexamethyldisilazane and aldehyde derivative VI to produce olefin VII. The olefin VII is treated with thiol derivative VIII in the presence of a suitable condensation reagent such as AlCl₃ to product adduct IX.

Cyano derivative IX is treated with a suitable reducing agent preferably SnCl₂/HCl to produce aldehyde derivative X. Quinaldine derivative III is treated with Ph₃P in a suitable solvent such as acetonitrile to form Wittig reagent XI, which may be reacted with a base such as butyl lithium and the aldehyde X to produce XII.

### METHOD B

Alternatively phosphonium salt V is treated with a strong base such as BuLi or the potassium salt of hexamethyldisilazane and aldehyde derivative XIII to produce olefin XIV. The olefin XIV is treated with thiol derivative VIII in the presence of a suitable condensation reagent such as AlCl₃ to produce adduct XV. Thiol adduct XV is treated with a reagent such as BBr₃ or AICI₃/EtSH to afford phenol XVI. The phenol derivative XVI is then reacted with a quinaldine derivative of general structure III in the presence of a suitable base such as NaOH, NaH, K₂C0₃ in an inert solvent such as THF, dioxane, DMF, etc., with warming if necessary to provide adducts XVII.

### METHOD C

Grignard reagent XVIII is reacted with aldehyde XIX to give alcohol XX which is oxidized with a suitable reagent such as CrO₃ to give ketone XXI. Phosphonium salt Va is treated with a strong base such as BuLi and ketone derivative XXI to produce olefin XXII. The double bond in XXII is reduced with hydrogen gas and a catalyst such as Pd on C to give saturated derivative XXIII. The derivative XXIII is treated with a reagent such as BBr₃ or AlCl₃/EtSH to afford phenol XXIV. The phenol derivative is then reacted with a quinaldine derivative of general structure III in the presence of a suitable base such as K₂C0₃ in an inert solvent such as THF with warming if necessary to provide adducts XXV.

### METHOD D

Bromo derivative XXVI is treated with Mg in a suitable solvent such as THF to afford the Grignard derivative XXVII. Aldehyde XIX is treated with the Grignard reagent XXVII to produce alcohol XXVIII which is oxidized with Cr03 to afford ketone XXIX. Phosphonium salt Va is treated with a strong base such as BuLi and ketone derivative XXIX to produce olefin XXX. The double bond is reduced with hydrogen gas using a suitable catalyst such as Pt on C to afford saturated derivative XXXI. Derivative XXXI is treated with (Bu)₄NF in THF to produce alcohol XXXII. Alcohol XXXII is oxidized with a suitable oxidant such as Mn0₂ to produce aldehyde XXXIII. Wittig reagent XI is reacted with a base such as butyl lithium and the aldehyde XXXIII to produce XXXIV. In Method D, the abbreviation PG represents the t-butyldiphenylsilyl group.

Structures XII, XVII, XXV and XXXIV are representatives of the Formula I compounds. In cases where Q¹ and Q² is an ester group, such esters may be hydrolyzed in a mixture of a polar solvent such as tetrahydrofuran (THF) and a strong base such as aqueous sodium hydroxide to provide the respective salts, acidification of which provides the corresponding acids. The salts and acids are also representatives of structure I. The invention is further defined by reference to the following examples, which are intended to be illustrative and not limiting.

In Table 1 are shown some representative Formula I compounds.

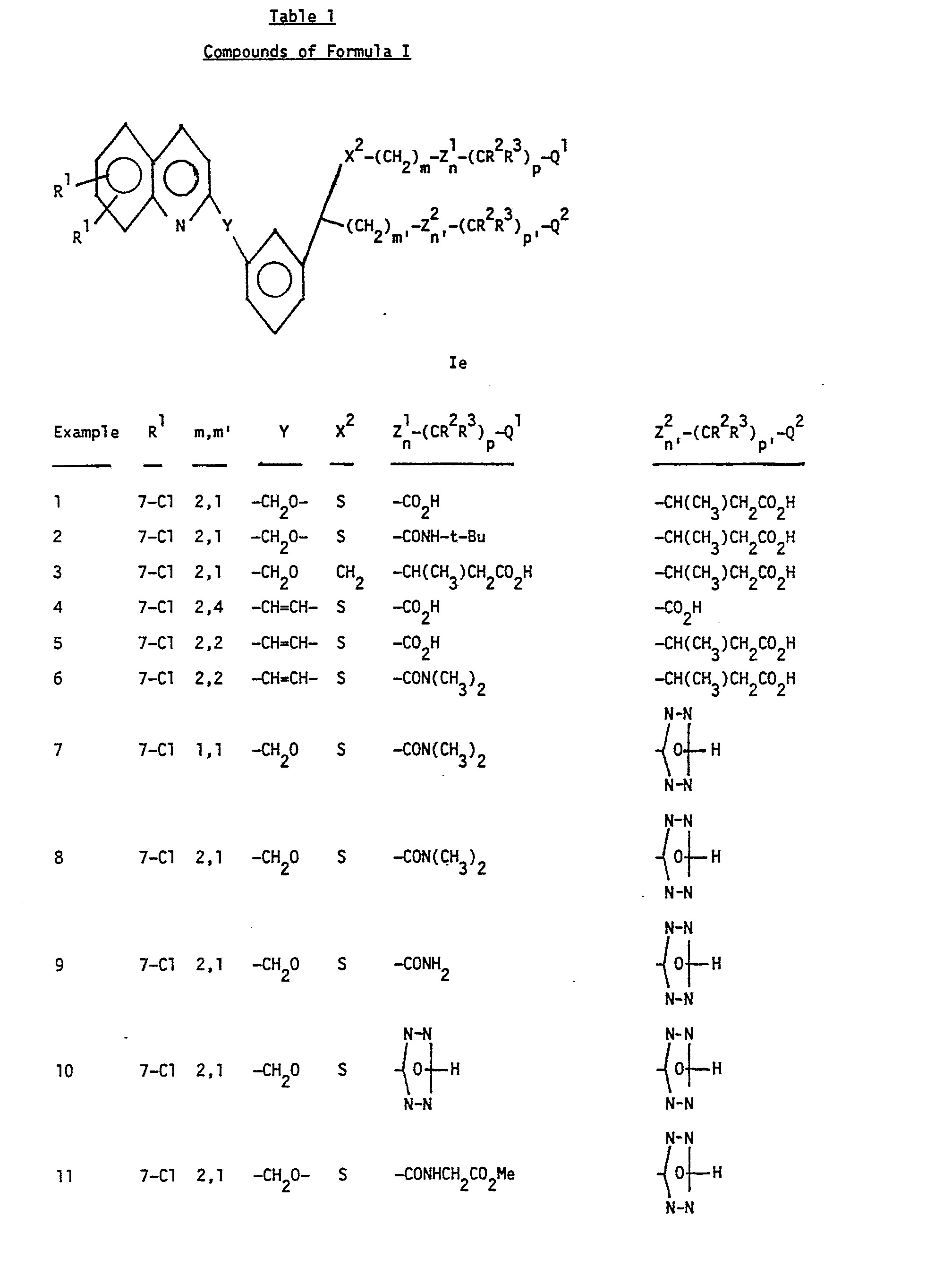

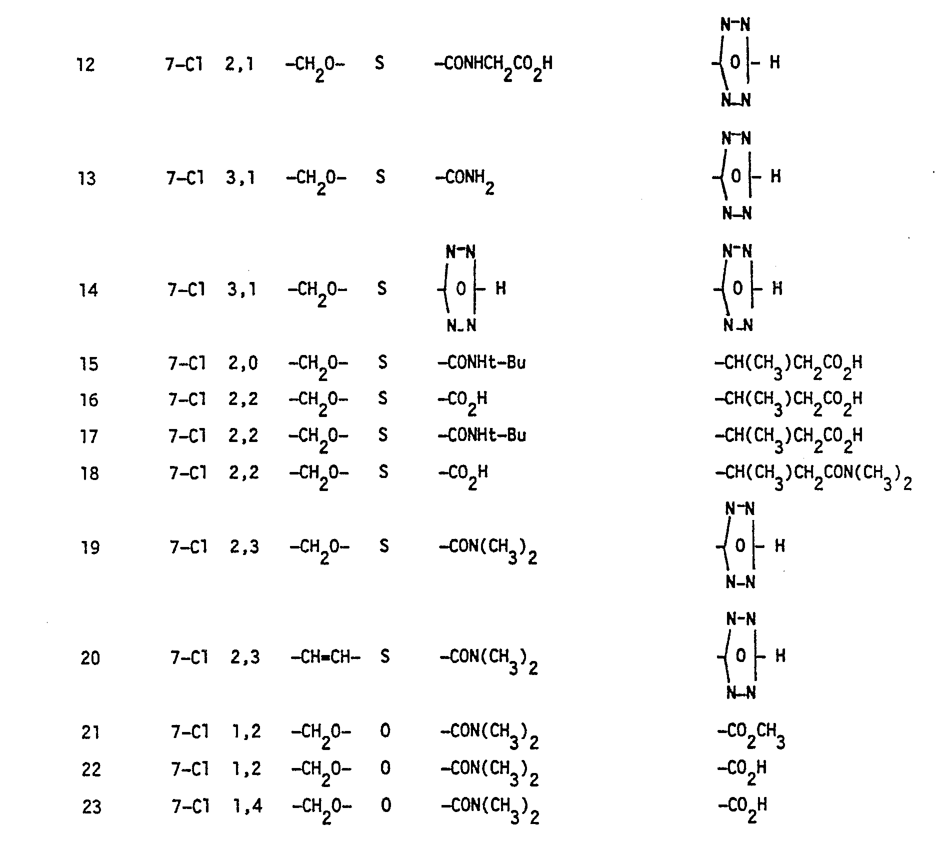

### Preparation of Starting Materials

### Preparation 1

### Preparation of 2-bromomethyl-7-chloroquinoline

A solution of 7-chloroquinaldine (177 g, 1 mole) N-bromosuccinimide (178 g, 1 mole), benzoylperoxide (1 g) in 2 L CCl₄ were heated at reflux for 2 days under a sun lamp. The reaction mixture was cooled, and passed through a plug of Si0₂ (~ 1 Kg) using toluene as eluent. Chromatography on 2 x 1 kg Si0₂ columns using toluene as eluent afforded 110-120 g of the title compound

m.p. 112°(d), p.m.r. (CDCl₃) 8: 8.3 (d,1H), 8.1-7.9 (m,2H), 7.4-7.7 (m,2H), 4.7 p.p.m. (s,2H)

### Preparation 2

### Preparation of (7-chloroquinolin-2-yl) methyltriphenylphosphonium bromide

To a suspension of 2-bromomethyl-7-chloroquinoline (120 g, .5 M) in 800 mL CH₃CN at 60° was added triphenylphosphine (183 g). The reaction mixture was heated overnight at 60°, cooled and 400 mL ether was added. The solid was filtered and dried to yield 170 g phosphonium salt.

p.m.r. (CDCI₃) 8: 7.3-8.2 (m,20 H), 6.0 p.p.m. (d,2H).

### EXAMPLE 1

### 5-(3-(7-Chloroquinolin-2-ylmethoxy)phenyl)-3-methyl-6-thianonane-1,9-dioic acid

### Step 1 Preparation of m-(t-butyldimethylsilyloxy) benzaldehyde

To a solution of m-hydroxybenzaldehyde (10 g) in dichloromethane (50 mL) was added t-butyldimethylsilyl chloride (15 g) followed by triethylamine (15 mL) and few crystals of dimethylaminopyridine. The reaction was stirred at room temperature for 48 hours. The reaction was quenched by addition of NH₄0Ac buffer and extracted with chloroform. The organic layer was washed once with water, dried over sodium sulfate and evaporated. The resulting residue was purified by flash chromatography using 100/0 ethyl acetate in hexane to afford the title compound, used as such in the next step.

### Step 2 Preparation of 1-(3-(t-butyldimethylsilyloxy) phenyl)-3-butene-2-one

To a solution of the benzaldehyde (10 g) from Step 1 in THF (100 mL) was added 1-triphenylphosphorany- lidene-2-propanone (31 g). The reaction was heated at 70° for 48 hours. The reaction was allowed to cool to room temperature and the solvent was removed by evaporation. The resulting residue was purified by flash chromatography using 20% ethyl acetate in hexane to afford the title compound, used as such in the next step.

### Step 3 Preparation of methyl 5-(3-(t-butyldimethylsilyloxy)phenyl)-7-oxo-4-thiaoctanoate

To a solution of the a, -unsaturated ketone prepared in step 2 (2.55 g) in THF (10 mL) was added methyl 3-mercaptopropionate (1.55 mL) followed by 1,5-diazabicyclo[4.3.0]non-5-ene (0.6 mL). The reaction was stirred at room temperature for 1 hour and it was evaporated to dryness. The resulting residue was purified by flash chromatography using 15% ethyl acetate in hexane to afford the title compound.

p.m.r. ((CD₃)₂CO) 0.2 (s, 6H), 1.0 (s, 9H), 2.1 (s, 3H), 2.4 to 2.6 (m, 4H), 3.0 (m, 2H), 3.6 (s, 3H), 4.4 (t, 1H), 6.8 (dd, 1H), 6.95 (t, 1H), 7.05 (d, 1H), 7.2 p.p.m. (t, 1H).

### Step 4 Preparation of 5-(3-(t-butyldimethylsilyloxy)phenyl)-3-methyl-6-thianon-2-ene-1,9-dioic acid, 1-ethyl, 9-methyl diester

To a solution of diisopropylamine (1.53 mL) in THF (22 mL) at -30° was added a solution of butyllithium (6.8 mL of 1.6M in hexane). After 30 min at -30° methyl (trimethylsilyl)acetate (2.0 mL) was slowly added and the reaction was cooled to -78° for 30 min. The reaction was transfered with a cannula to a solution of the ketone prepared in step 3 (2.88 g) in THF (22 mL) at -78°. After 15 min at -78°, the reaction was allowed to warm to room temperature over 30 min.

The reaction was quenched with NH₄0Ac buffer, extracted with ethyl acetate, dried over sodium sulfate and evaporated. The resulting residue was purified by flash chromatography using 100/0 ethyl acetate in hexane to afford the title compound as a colorless oil.

p.m.r. ((CD₃)₂CO) 0.2 (s, 6H), 1,0 (s, 9H), 1.2 (2t, 3H), 1.8 and 2.05 (2d, 3H, methyl on E and Z isomers), 2.4 to 2.7 (m, 5H), 3.1 to 3.3 (m, 1 H), 3.6 (s, 3H), 4.05 (2q, 2H), 4.2 (2t, 1 H), 5.6 (2 bt, 1 H, olefinic hydrogen on E and Z isomers), 6.8 (dd, 1H), 6.95 (bs, 1H), 7.05 (m, 1H), 7.2 p.p.m. (2t, 1H).

### Step 5 Preparation of 5-(3-(t-butyldimethylsilyloxy)phenyl)-3-methyl -6-thainonane-1,9-dioic acid, 1-ethyl, 9-methyl diester

To a solution of the a, -unsaturated ester (1.01 g) from step 4 in ethyl acetate (50 mL) was added platinum oxide (305 mg). The resulting brown suspension was shaken in a Parr hydrogenator under 45 psi of hydrogen for 66 hours. Chloroform (25 mL) was added to the black suspension and the reaction was filtered twice through silica gel pads. The solvents were removed by evaporation, and the resulting residue was purified by flash chromatography using 10% ethyl acetate in hexane to afford a mixture of compounds of the same Rf. Analytical HPLC showed a mixture of 4 compounds, of which 2 are starting materials E and Z olefins left unreacted. Of the two remaining products, the major one (about 1 to 10) was isolated by successive injection on an HPLC using a ZORBAX sil 21 mm x 25 cm column at a flow rate of 22 mL/min with 5% ethyl acetate in hexane to afford the title compound.

p.m.r. ((CD₃)₂CO) 0.2 (s, 6H), 0.9 (d, 3H), 1.0 (s, 9H), 1.2 (t, 3H), 1.6 to 2.2 (m, 4H), 2.35 to 2.6 (m, 5H). 3.6 (s, 3H), 4.0 (t, 1H), 4.05 (q, 2H), 6.75 (dt, 1H), 6.95 (t, 1H), 7.0 (dt, 1H), 7.2 p.p.m. (t, 1H).

### Step 6 Preparation of 5-(3-hydroxyphenyl)-3-methyl-6-thianonane-1,9-dioic acid, 1-ethyl, 9-methyl diester

To a solution of the silyl ether from step 5 (192 mg) in THF (4 mL) was added glacial acetic acid (90 µL) followed by a solution of tetra-n-butylammonium fluoride (0.81 mL of 1M in THF). The reaction was stirred at room temperature for 40 minutes. Ammonium acetate buffer solution was added to the reaction and it was extracted with ethyl acetate. The organic layer was dried over sodium sulfate and evaporated. The resulting residue was purified by flash chromatography using 40% ethyl acetate in hexane to afford the title compound. p.m.r. ((CD₃)₂CO) 0.95 (d, 3H), 1.2 (t, 3H), 1.6 to 1.9 (m, 2H), 2.0 to 2.15 (m, 1H), 2.3 to 2.6 (m, 6H), 3.6 (s, 3H), 3.95 (t, 1H), 4.1 (q, 2H), 6.7 (dt, 1H), 6.85 (m, 2H), 7.15 p.p.m. (t, 1H).

### Step 7 Preparation of5-(3-(7-chloroquinolin-2-ylmethoxy)phenyl)-3-methyl-6-thianone-1,9-dioic acid,1-ethyl,

### 9-methyl diester

To a solution of the phenol from step 6 (140 mg), 2-bromomethyl-7-chloroquinoline (140 mg) from preparation 1 in acetone (2.8 mL) was added milled potassium carbonate (110 mg). The reaction was refluxed for 3.5 hours. After cooling to room temperature, ethyl acetate (10 mL) was added to the reaction and the resulting suspension was filtered. The solvents were removed by evaporation and the resulting residue was purified by flash chromatography using 30% diethyl ether in hexane to afford the title compound. p.m.r. ((CD₃)₂CO) 0.85 (d, 3H), 1.2 (t, 3H), 1.6 to 2.1 (m, 4H), 2.3 to 2.5 (m, 5H), 3.6 (s, 3H), 4.0 (q+t, 3H), 5.4 (s, 2H), 7.0 (td, 2H), 7.15 (t, 1H), 7.3 (t, 1H), 7.6 (dd, 1 H), 7.75 (d, 1H), 8.0 (d, 1 H), 8.05 (d, 1H), 8.4 p.p.m. (d, 1H).

### Step 8

To a solution of the diester from step 7 (147 mg) in 1,2-dimethoxyethane (3.5 mL) was added an aqueous solution of lithium hydroxide (1.5 mL of 1 N). The reaction was stirred for 10 hours at room temperature. Water was added and the reaction was extracted with ethyl acetate. The aqueous layer was acidified to pH 3 with HCI (2N) and extracted twice with ethyl acetate. The organic layer was dried over sodium sulfate and evaporated. The resulting residue was dried under high vacuum and it solidified. Recrystallization of the solid in acetone/hexane afforded the title compound.

m.p. 155-157°C p.m.r. ((CD₃)₂CO) 0.9 (d, 3H), 1.6 to 2.1 (m, 4H), 2.4 to 2.6 (m, 5H), 4.1 (t, 1 H), 5.4 (s, 2H), 6.95 (dd, 1H), 7.0 (d, 1H), 7.2 (t, 1H), 7.3 (t, 1H), 7.6 (dd, 1H), 7.7 (d, 1H), 8.0 (d, 1H), 8.05 (d, 1H), 8.5 p.p.m. (d, 1H).

### EXAMPLE 2

### 8-(N-t-Butylcarbamyl)-5-(3-(7-chloroquinolin-2-ylmethoxy)phenyl)-3-methyl-b-thiaoctanoic acid

### Step 1 Preparation of 1-(3-(t-butyldiphenylsilyloxy)phenyl)-3-butene-2-one

Using the procedures described in Example 1, step 1 and step 2 but substituting t-butyldiphenylsilyl chloride for t-butyldimethylsilyl chloride in step 1 there was obtained the title compound used as such in the next step.

### Step 2 Preparation of 5-(3-(t-butyldipheny)silyloxy) phenyl)-7-oxo-4-thiaoctanoic acid

To a solution of the a, -unsaturated ketone from step 1 (3.0 g) in THF (8 mL) was added 3-mercaptopropionic acid (0.72 mL) followed by 1,5-diazabicyclo[4,3,O]non-5-ene (1.25 mL). The reaction was stirred at room temperature for 1.5 hours and it was evaporated to dryness. The resulting residue was purified by flash chromatography using 1% of acetic acid in a solution of 100/o acetone in toluene to afford the title compound.

p.m.r. ((CD₃)₂CO) 1.1 (s, 9H), 2.0 (s, 3H), 2.3-2.4 (m, 4H), 2.7 (d, 2H), 4.2 (t, 1H), 6.7 (dt, 1H), 6.8 (t, 1H), 6.9 (bd, 1H), 7.1 (t, 1H), 7.4 (m, 6H), 7.8 p.p.m. (m, 4H).

### Step 3 Preparation of 5-(3-(t-butyldiphenylsilyloxy)phenyl)-8-methoxycarbonyl-7-methyl-4-thia-7-octenoic acid

To a solution of diisopropylamine (6.75 mL) in THF (55 mL) at -30° was added a solution of butyllithium (30 mL of 1.6M in hexane). After 30 min at -30° methyl (trimethylsilyl)acetate (8.0 mL) was slowly added and the reaction was cooled to -78 for 30 min. The reaction was transfered with a cannula to a solution of ketone prepared in step 2 (9.6 g) in THF (55 mL) at -78°. After 1 hour at -78° the reaction was quenched with NH₄0Ac buffer. This mixture was extracted with ethyl acetate, dried over sodium sulfate and evaporated. The residue was purified by flash chromatography using 0.1 % of acetic acid in a 500/o solution of ether in hexane to afford the title compound.

p.m.r. ((CD₃)₂CO) 1.1 (s, 9H), 1.6, 2.1 (2d, 3H), 2.4-2.8 (m, 5H), 2.9, 3.2 (2dd, 1H), 3.6 (2s, 3H), 4.1 (m, 1H), 5.5, 5.6 (2bs, 1H, E and Z olefins), 6.7 (dt, 1H), 6.85 (td, 1H), 6.9 (bd, 1H). 7.1 (m, 1H), 7.4 (m, 6H), 7.8 (m, 4H).

### Step 4 Preparation of methyl

### 8-t-butylcarbamyl-5-(3-(t-butyldiphenylsilyloxy)phenyl)-3-methyl-6-thia-2-octenoate

To a solution of acid (step 3) (4.0 g), triethylamine (2.0 mL) in dichloromethane (34 mL) and acetonitrile (8 mL) at 0° was added 2-chloro-1-methylpyridinium iodide (3.7 g). The suspension was stirred 1 hour at 0° to afford a yellow solution. To this solution was added t-butylamine (1.5 mL) and the reaction was stirred at room temperature for 1 h. The mixture was evaporated and the residue was partitioned between ethyl acetate and water. The pH of the aqueous phase was adjusted to pH 4 before extraction. The organic phase was dried over sodium sulfate and evaporated. Flash chromatography of the residue using 40% of ether in hexane afforded the title compound.

p.m.r. ((CD₃)₂CO) 1.1 (s, 9H), 1.3 (2s, 9H), 1.6,2.0 (2d, 3H), 2.2-2.6 (m, 5H), 2.9, 3.2 (2m, 1H), 3.6 (2s, 3H), 4.1 (m, 1 H), 5.5, 5.6 (2bs, 1 H, E and Z olefins), 6.6 (b, 1 H), 6.65 (dt, 1 H), 6.85 (t, 1 H), 6.9 (dt, 1 H), 7.1 (m, 1 H), 7.4 (m, 6H), 7.7 p.p.m. (m, 4H).

### Step 5 Preparation of methyl 8-t-butylcarbamyl-5-(3-(t-butyldiphenylsiloxy)phenyl)-3-methyl-6-thiaoctanoate

A solution of the a, -unsaturated ester prepared in step 4 (2.0 g) and platinum oxide (500 mg) in ethyl acetate (100 mL) was shaken in a Parr hydrogenator for 14 hours under a pressure of 44 p.s.i. of hydrogen. Chloroform (50 mL) was added to the reaction and the catalyst was removed by filtration through a silica pad. The resulting black residue was purified by flash chromatography using 500/o ether in hexane. The compound obtained was hydrogenated a second time in the Parr in the same conditions. The compound was isolated and purified the same way from the black suspension. It was used as such in the next sep.

p.m.r. ((CD₃)₂CO) 0.8 (d, 3H), 1.1 (s, 9H), 1.3 (s, 9H), 1.3-1.8 (m, 3H), 2.1-2.4 (m, 6H), 3.6 (s, 3H), 3.8 (2d, 1H), 6.6 (b, 1H), 6.7 (td, 1H), 6.8 (t, 1H), 6.9 (dt, 1H), 7.1 (t, 1H), 7.4 (m, 6H), 7.7 p.p.m. (m, 4H).

### Step 6

Using the procedures described in Example 1 from step 6 to 8 and substituting the silyl ether obtained in step 5 for the 5-(3-(t-butyldimethylsilyloxy)phenyl)-3-methyl-6-thianonane-1,9-dioic acid, 1-ethyl, 9-methyl diester of Example 1 in step 6, the title compound was obtained.

p.m.r. ((CD₃)CO) 0.9 (d, 3H), 1.3 (s, 9H), 1.6-2.0 (m, 3H), 2.1-2.5 (m, 6H), 4.0 (t, 1H), 5.4 (s, 2H), 6.7 (bs, 1H), 6.9-7.0 (m, 2H), 7.15 (t, 1H), 7.3 (t, 1H), 7.6 (dd, 1H), 7.75 (d, 1 H), 8.0 (d, 1H), 8.05 (d, 1H), 8.4 p.p.m. (d, 1H).

### EXAMPLE 3

### 5-(3-(7-Chloroquinolin-2-ylmethoxy)phenyl)-3,7-dimethylnonane-1,9-dioic acid

### Step 1 Preparation of methyl 3-methyl-7-oxooct-5[E]-enoate

To a solution of (±) methyl 3-methyl-5-oxopentanoate (2.0 g) (prepared by the method of P.G. Williard, J. Am. Chem. Soc. 1985, 107, 199-203) in THF (70 mL) was added 1-triphenylphospharanylidene-2-propanone (5.3 g). The reaction was stirred at 60° for 24 hours. The reaction was allowed to cool to room temperature and the solvent was removed by evaporation. The residue was purified by flash chromatography using 400/0 diethyl ether in hexane to afford the title compound.

p.m.r. ((CD₃)₂CO) 0.95 (d, 3H), 2.2 (s, 3H), 2.1 to 2.6 (m, 5H), 3.6 (s, 3H), 6.0 (d, 1H), 6.8 p.p.m. (dt, 1H).

### Step 2 Preparation of the t-butyldimethylsilyl ether of m-bromophenol

To a solution of m-bromophenol (5.0 g) in dichloromethane (35 mL) was added triethylamine (3.3 mL) followed by t-butyldimethylsilyl chloride (3.6 g). The reaction was stirred at room temperature for 24 hours and it was quenched with NH₄0Ac buffer. The product was extracted with ethyl acetate, it was washed once with water, dried over sodium sulfate and evaporated. The resulting residue was purified by flash chromatography using 8% ethyl acetate in hexane to afford the title compound, used as such in the next step.

Step 3 Preparation of a solution of 3-(t-butyldimethylsilyloxy)phenylmagnesium bromide 1M in THF Magnesium turnings (49 mg) in a round bottom flask were flame dried under a stream of nitrogen. After cooling to room temperature, THF (1 mL) was added followed by a solution of t-butyldimethylsilyl ether of m-bromophenol (575 mg) in THF (1 mL). The reaction was stirred at room temperature for 2.5 hours to give a light grey-green solution which was used as such in the next step.

### Step 4 Preparation of methyl 5-(3-(t-butyldimethylsilyloxy)phenyl)3-methyl-7-oxooctanoate

To a suspension of copper (I) cyanide (80 mg) in THF (0.7 mL) at -78° was slowly added a solution of 3-(t-butyldimethylsilyloxy)phenyl magnesium bromide 1M in THF (1.65 mL). The dry-ice bath was replaced by an ice-water bath. The reaction was stirred at 0° for 6 minutes and the ice-water bath was replaced by the dry-ice bath. A solution of methyl 3-methyl-7-oxooct-5[E]-enoate (100 mg) in THF (0.5 mL) was slowly added to the reaction at -70°. The reaction was stirred for 20 min and quenched with a solution of 10% V/V of concentrated ammonium hydroxide in aqueous ammonium chloride saturated. The reaction was extracted with diethyl ether, dried over sodium sulfate and evaporated. The resulting residue was purified by flash chromatography using 25% diethyl ether in hexane to afford the title compound.

p.m.r. ((CD₃)₂CO) 0.2 (s, 6H), 0.9 (d, 3H), 0.95 (s, 9H), 1.4 (m, 1H), 1.65 (m, 2H), 2.0 (s, 3H), 2.0 to 2.2 (m, 2H), 2.7 (d, 2H), 3.2 (m, 1H), 3.5 (s, 3H), 6.7 (dt, 1H), 6.75 (t, 1H), 6.85 (bd, 1H), 7.2 p.p.m. (t, 1H).

### Step 5 Preparation of 1-ethyl 9-methyl 5-(3-(t-butyldimethylsilyloxy)phenyl-3,7-dimethylnon-2-ene-1,9-dioate

To a solution of diisopropylamine (92 ilL) in THF (1.2 mL) at -30° was added a solution of butyllithium (0.44 mL of 1.5M in hexane). After 1 hour at -30° C the reaction was cooled to -78° and ethyl (trimethylsilyl)acetate (125 µL) was added dropwise. After 1 hour at -78° the reaction was transferred with a cannula to a solution of methyl 5-(3-(t-butyldimethylsilyloxyjphenyl)-3-methyl-7-oxooct anoate (77 mg) in THF (0.6 mL) at -78°. After 1 hour at -78° the reaction was allowed to stir at room temperature for 30 minutes. The reaction was quenched with NH₄0Ac buffer, extracted with ethyl acetate, dried over sodium sulfate and evaporated. The resulting residue was purified by flash chromatography using 200/o diethyl ether in hexane to afford the title compound as a 1 to 1 mixture of E and Z olefins.

p.m.r. ((CD₃)₂CO) 0.2 (d, 6H), 0.9 (t, 3H), 1.0 (s, 9H), 1.2 (dt, 3H), 1.4 (m, 1 H), 1.6 to 1.8 (m, 2H), 1.7 and 2.1 (2d, 3H, CH₃ on E and Z olefins), 2.1 to 2.25 (m, 2H), 2.35 to 2.5 (m, 2H), 3.0 to 3.1 (m, 1 H), 3.55 (s, 3H), 4.05 (dq, 2H), 5.5 and 5.6 (2bs, 1H, H on E and Z olefins), 6.7 (dd, 1H), 6.75 (t, 1H), 6.85 (dd, 1H), 7.15 p.p.m. (dt, 1H).

### Step 6 Preparation of ethyl methyl 5-(3-(t-butyldimethylsilyloxy)phenyl)-3,7-dimethylnonane-1.9-dioate

To a solution of the olefin (219 mg) from step 5 in ethyl acetate (5.5 mL) was added platinum oxide (5 mg). The resulting suspension was stirred under a hydrogen atmosphere for 1 hour at room temperature. The reaction was filtered on celite and evaporated to afford the title compound which was used as such in the next step.

p.m.r. ((CD₃)₂CO) 0.2 (s, 6H), 0.85 to 0.95 (3d, 6H), 1.0 (s, 9H), 1.15 (2t, 3H), 1.3 to 1.9 (m, 6H), 2.0 to 2.4 (m, 4H), 2.8 (m, 1 H), 3.6 (s, 3H), 4.0 (2q, 2H), 6.7 (m, 2H), 6.8 (dt, 1 H), 7.2 p.p.m. (t, 1 H).

### Step 7 Preparation of ethyl methyl 3,7-dimethyl-5-(3-hydroxyphenyl)nonane-1,9-dioate

To a solution of the silyl ether from step 6 (261 mg) in THF (5 mL) was added glacial acetic acid (120 µL) followed by a solution tetra-n-butylammonium fluoride (1.1 mL of 1 M in THF). The reaction was stirred at room temperature for 45 minutes. Ammonium acetate buffer solution was added to the reaction and it was extracted with ethyl acetate. The organic layer was dried over sodium sulfate and evaporated. The resulting residue was purified by filtration through a silica gel pad washed with ethyl acetate. Evaporation to dryness afforded the title compound which was used as such in the next step.

p.m.r. 0.85 to 0.95 (3d, 6H), 1.1 to 1.25 (2t, 3H), 1.3 to 1.7 (m, 6H), 2.0 to 2.4 (m, 4H), 2.8 (m, 1 H), 3.6 (s, 3H), 4.1 (2q, 2H), 6.7 (m, 3H), 7.1 (t, 1H), 8.2 p.p.m. (s, 1H).

### Step 8 Preparation of ethyl methyl 5-(3-(7-chloroquinolin-2-ylmethoxy)phenyl)-3,7-dimethytnonane-1,9-dioate

To a solution of the phenol from step 7 (136 mg), 2-bromomethyl-7-chloroquinoline (140 mg) from preparation 1 in acetone (2.8 mL) was added milled potassium carbonate (111 mg). The reaction was refluxed for 5 hours. After cooling to room temperature, ethyl acetate (5 mL) was added to the reaction and the resulting suspension was filtered. The solvents were removed by evaporation and the resulting residue was purified by flash chromatography using 350/o of diethyl ether in hexane to afford the title compound. p.m.r. ((CD₃)₂CO) 0.8 to 0.9 (3d, 6H), 1.1 to 1.2 (2t, 3H), 1.3 to 1.8 (m, 6H), 2.0 to 2.4 (m, 4H), 2.8 (m, 1 H), 3.6 (s, 3H), 4.0 (2t, 2H), 5.4 (s, 2H), 6.8 to 7.0 (m, 3H), 7.2 (t, 1H), 7.6 (dd, 1H), 7.7 (d, 1H), 8.0 (d, 1H), 8.05 (d, 1H), 8.4 p.p.m. (d, 1H).

### Step 9

To a solution of the diester prepared in step 8 (188 mg) in 1,2-dimethoxy ethane (4.5 mL) was added an aqueous solution of lithium hydroxide (2.7 mL of 1N). The reaction was stirred for 24 hours at room temperature. Water was added and the reaction was extracted with ethyl acetate. The aqueous layer was acidified to pH 3 with HCI 2N and extracted twice with ethyl acetate. The organic layer was dried over sodium sulfate and evaporated. The resulting residue was purified by flash chromatography using 1% of acetic acid in a solution of 100/₀ of THF in toluene to afford the title compound.

p.m.r. ((CD₃)₂CO) 0.8 to 0.95 (2d, 6H). 1.4 to 1.9 (m, 6H), 2.0 to 2.3 (m, 4H), 2.8 (m, 1 H), 5.4 (s, 2H), 6.8 (td, 2H), 7.0 (m, 1H), 7.2 (t, 1H), 7.6 (dd, 1H), 7.7 (d, 1H), 8.0 (d, 1H), 8.05 (d, 1H), 8.4 p.p.m. (d, 1H).

### EXAMPLE 4

### 5-(3-(2-(7-Chloraquinolin-2-yl)ethenyl)phenyl)-4-thiadecane-1,10-dioic acid

### Step 1 Preparation of 6-(3-bromophenyl)hex-5-enoic acid

To a suspension of (4-carboxybutyl) triphenylphosphonium bromide (5.6 g) in THF (100 mL) at 0° was slowly added a solution of potassium hexamethyldisilazane (42 mL of 0.67M) in toluene. After 30 min a solution of m-bromobenzaldehyde (2.0 g) in THF (5 mL) was slowly added. The reaction was stirred 1.5 h at room temperature and it was quenched with NH₄0Ac buffer. The aqueous phase was acidified with 2N hydrochloric acid until pH 5. The reaction was extracted with ethyl acetate, dried over sodium sulfate and evaporated. The residue was purified by flash chromatography using 1% of acetic acid in 350/o of ethyl acetate in hexane to afford the title compound.

p.m.r. ((CD₃)₂CO) 1.8 (m, 2H), 2.3 (m, 4H), 5.8 6.4 (2m, 2H, E and Z olefins), 7.1-7.6 (m, 4H), 10.5 p.p.m. (bs, 1H).

### Step 2 Preparation of methyl 6-(3-bromophenyl) hex-5-enoate

To a solution of the acid from step 1 (2.49 g) in methanol (50 mL) was added concentrated sulfuric acid (0.5 mL). The reaction was stirred at room temperature for 4 hours and the total volume of the reaction was reduced to approximately 5 mL by evaporation. The resulting liquid was partitioned between ethyl acetate and a solution of aqueous sodium bicarbonate. The organic layer was washed once with water, dried over sodium sulfate and evaporated to afford the title compound.

p.m.r. ((CD₃)₂CO) 1.8 (m, 2H), 2.3 (m, 4H), 3.6 (2s, 3H), 5.7, 6.4 (2m, 2H, E and Z olefins), 7.2-7.6 p.p.m. (m, 4H).

### Step 3 Preparation of dimethyl 5-(3-bromophenyl)-4-thiadecane-1,10-dioate

To a solution of the styrene from step 2 (2.48 g) and methyl 3-mercaptopropionate (2.0 mL) in cyclohexane (45 mL) was added dropwise titanium tetrachloride (5.0 mL). The reaction was mechanically stirred for 8 hours and left at room temperature in the dark for 3 days. The reaction was carefully quenched with NH₄0Ac buffer at 0°C, hydrochloric acid 2N was added until pH 4 and ethyl acetate was used to extract the compound. The organic layer was dried over sodium sulfate and evaporated. The residue was purified by flash chromatography using 30% of ether in hexane to afford the title compound.

p.m.r. ((CD₃)₂CO) 1.2-1.7 (m, 4H), 1.8-1.9 (m, 2H), 2.3 (t, 2H), 2.5 (m, 4H), 3.6 (2s, 6H), 3.9 (t, 1 H), 7.3 (t, 1H), 7.4 (m, 2H), 7.6 p.p.m. (t, 1H).

### Step 4 Preparation of dimethyl 5-(3-cyanophenyl)-4-thiadecane-1,10-dioate

To a solution of the bromo compound from step 3 (1.02 g) in DMF (8.5 mL) was added copper (I) cyanide (680 mg). The reaction was refluxed for 6 hours, allowed to cool to room temperature and poured onto a solution of 100/0 of concentrated ammonium hydroxide in a saturated aqueous solution of ammonium chloride. The mixture was extracted with ether, the organic layer was dried over sodium sulfate and evaporated. The residue was purified by flash chromatography using 50% of ether in hexane to afford the title compound. p.m.r. ((CD₃)₂CO) 1.2-1.7 (m, 4H), 1.8-1.9 (m, 2H), 2.3 (t, 2H), 2.5 (m, 4H), 3.6 (2s, 6H), 4.0 (t, 1H), 7.6 (t, 1H), 7.65 (td, 1H), 7.75 (td, 1H), 7.8 p.p.m. (t, 1H).

### Step 5 Preparation of dimethyl 5-(3-formylphenyl)-4-thiadecane-1,10-dioate

To a suspension of tin (II) chloride anhydrous (1.85 g) in diethyl ether (8 mL) with gentle stirring at room temperature was bubbled gaseous hydrochloric acid until no more solid tin chloride remains and two liquid phases are obtained. A solution of nitrile from step 4 (420 mg) in ether (2.0 mL) was added to the vigorously stirred reaction. Gaseous hydrochloric acid was bubbled in the reaction for 30 min. The reaction was stirred vigorously for 3 hours and water (1.5 mL) was carefully added. The mixture was stirred for 30 min and extracted (2x) with ethyl acetate. The combined extracts were washed once with water, dried over sodium sulfate and evaporated. The residue was purified by flash chromatography using 80/₀ ethyl acetate in hexane to afford the title compound.

p.m.r. ((CD₃)₂CO) 1.2-1.7 (2m, 4H), 1.9 (m, 2H), 2.2 (t, 2H), 2.5 (m, 4H), 3.6 (2s, 6H), 4.1 (t, 1H), 7.6 (t, 1H), 7.7 (dt, 1H), 7.8 (dt, 1H), 7.9 (t, 1H), 10.1 p.p.m. (s, 1H).

### Step 6 Preparation of dimethyl 5-(3-(2-(7-chloroquinoJin-2-yl)ethenyl)phenyl)-4-thiadecane-1,10-dioate

To a suspension of (7-chloroquinolin-2-yl)methyltriphenylphosphonium bromide from preparation 2 (260 mg) in THF (4 mL) at -78° was added dropwise a solution of n-butyllithium (0.32 mL of 1.55M in hexane). The reaction mixture was stirred 20 min and a solution of aldehyde from step 5 (175 mg) in THF (1.5 mL) at -78° was transferred dropwise to the reaction through a cannula. The reaction was stirred 15 min at -78° and 45 min at room temperature. The reaction was quenched with NH₄0Ac buffer, extracted with ethyl acetate, dried over sodium sulfate and evaporated. The residue was purified by flash chromatography using 15% of ethyl acetate in hexane to afford the title compound.

p.m.r. ((CD₃)₂CO) 1.2-1.7 (2m, 4H), 1.9 (q, 2H), 2.2 (t, 2H), 2.5 (m, 4H), 3.6 (2s, 6H), 4.0 (t, 1 H), 7.4-7.55 (m, 4H), 7.6 (dt, 1H), 7.7 (bs, 1H), 7.8-8.0 (m, 4H), 8.3 p.p.m. (d, 1H).

### Step 7

To a solution of diester from step 6 (100 mg) in 1,2-dimethoxyethane (2.5 mL) was added an aqueous solution of lithium hydroxide (0.5 mL of 1N). The reaction was stirred at room temperature for 19 hours. Water (5 mL) and sodium hydroxide (0.5 mL of 2N) were added and the mixture was washed with ethyl acetate. The aqueous layer was acidified with hydrochloric acid 2N until pH 3 and extracted with ethyl acetate (2x). The combined extracts were dried over sodium sulfate and evaporated. The residue was purified by flash chromatography using 1% of acetic acid in 150/0 of acetone in toluene to afford the title compound. p.m.r. ((CD₃)₂CO) 1.3-1.7 (m, 4H), 1.9 (q, 2H), 2.3 (t, 2H), 2.5 (m, 4H), 4.0 (t, 1 H), 7.4-7.55 (m, 4H), 7.65 (m, 1H), 7.8 (bs, 1H), 7.9-8.0 (m, 4H), 8.3 p.p.m. (d, 1H).

### EXAMPLE 5

### 5-(3-(2-(7-Chloroquinolin-2-yl)ethenyl)phenyl)-8-methyl-4-thiadecane-1,10-dioic acid

### Step 1 Preparation of methyl 6-(3-bromophenyl)-3-methylhex-5-enoate

To a suspension of (4-methoxycarbonyl-3-methylbutyl)triphenylphosphonium bromide from Example 16, step 5 (6.74 g) in HMPA (15 mL) and THF (100 mL) at -78° was slowly added a solution of potassium hexamethyldisilazane (20 mL of 0.65M) in toluene. After 30 min a solution of m-bromobenzaldehyde (2.19 g) in THF (7 mL) at -78° was slowly added through a cannula. The reaction was stirred for 30 min at -10° and 1 h at room temperature. The reaction was quenched with NH₄0Ac buffer, extracted with ethyl acetate, dried over sodium sulfate and evaporated. The residue was purified by flash chromatography using 100/o of ether in hexane to afford the title compound.

p.m.r. ((CD₃)₂CO) 0.95 (d, 3H), 2.1-2.4 (m, 5H), 3.6 (s, 3H), 5.8 (dt, 1H), 6.5 (dt, 1H), 7.3 (m, 2H), 7.4 p.p.m. (m, 2H).

### Step 2 Preparation of dimethyl 5-(3-bromophenyl)-8-methyl-4-thiadecane-1,10-dioate

To a solution of styrene from step 1 (2.5 g) and methyl 3-mercaptopropionate (1.9 mL) in dichloromethane (75 mL) at -10° was added aluminium chloride (5.7 g) in 3 portions. The reaction was stirred at room temperature for 45 min and carefully quenched with NH₄0Ac buffer. The mixture was extracted with ethyl acetate, dried over sodium sulfate and evaporated. The residue was purified by flash chromatography using 15% of ethyl acetate in hexane to afford the title compound.

p.m.r. ((CD₃)₂CO) 0.9 (2d, 3H), 1.1-1.5 (m, 2H), 1.8-2.0 (m, 3H), 2.1-2.3 (m, 2H), 2.5 (m, 4H), 3.6 (2s, 6H), 3.9 (t, 1H), 7.3 (t, 1H), 7.35-7.45 (m, 2H), 7.6 p.p.m. (t, 1H).

### Step 3

Using the procedure described in Example 4, step 4 to 7, but substituting dimethyl 5-(3-bromophenyl)-8-methyl-4-thiadecane-1,10-dioate for dimethyl 5-(3-bromophenyl)-4-thiadecane-1,10-dioate in step 4 there was obtained the title compound.

p.m.r. ((CD₃)₂CO) 0.9 (2d, 3H), 1.2-1.6 (m, 2H), 1.9-2.0 (m, 3H), 2.3 (m, 2H), 2.5 (m, 4H), 4.0 (t, 1 H), 7.4-7.5 (m, 4H), 7.6 (m, 1H), 7.7-8.0 (m, 5H), 8.3 p.p.m. (d, 1H).

### EXAMPLE 6

### 9-Dimethylcarbamyl-6-(3-(2-(7-chloroquinolin-2-yl)ethenyl)phenyl)-3-methyl-7-thianonanoic acid

Using the procedure described in Example 5 but substituting N,N-dimethyl 3-mercaptopropionamide for methyl 3-mercaptopropionate in step 2 there was obtained the title compound.

p.m.r. ((CD₃)₂CO) 0.9 (2d, 3H), 1.2-1.6 (m, 2H), 1.9-2.0 (m, 3H), 2.3 (m, 2H), 2.5 (m, 4H), 2.8, 2.9 (2s, 6H), 4.0 (t, 1H), 7.4-7.5 (m, 4H), 7.6 (m, 1H), 7.7 (bs, 1H), 7.8-8.0 (m, 4H), 8.3 p.p.m. (d, 1H).

### EXAMPLE 7

### N,N-Dimethyl 4-(3-(7-chloroquinolin-2-ylmethoxy)phenyl)-5-(1 H-tetrazol-5-yl)-3-thiapentanamide

### Step 1 Preparation of 3-(7-chloroquinolin-2-ylmethoxy)benzaldehyde

3-Hydroxybenzaldehyde (4.34 g, 35.5 mmoles), 2-bromomethyl-7-chloroquinoline from preparation 1 (10.03 g, 1.1 equiv.), and milled K₂C0₃ (7.34 g, 1.5 equiv.) were mixed together in acetone (100 mL) and heated to reflux for 8 hours. Then, EtOAc (100 mL) was added, the reaction mixture filtered on celite and evaporated. Flash chromatography on silica using EtOAc:hexane 20:80 afforded the title compound.

¹H NMR (CD₃COCD₃) 8 5.47 (s, 2H), 7.42 (m, 1H), 7.53-7.63 (m, 4H), 7.78 (d, 1H), 8.00-8.08 (m, 2H), 8.43 (d, 1H), 10.00 (s, 1H), p.p.m.

### Step 2 Preparation of 3-(3-(7-chloroquinolin-2-ylmethoxy)phenyl)-2-propenenitrile

To cyanomethyl triphenylphosphonium chloride (9.176 g, 1.2 equiv.) in THF (100 mL) at -78°C, n-BuLi 1.6M in hexane (16 mL, 1.1 equiv.) was added dropwise. After 40 min of stirring at -78°C, the substituted benzaldehyde (step 1, 6.74 g, 22.6 mmoles) in 50 mL THF was added dropwise. When the addition was completed, the temperature was gradually increased to room temperature and the mixture stirred another hour. Hydrolysis with 25% aqueous NH₄0Ac, extraction with EtOAc evaporation and flash chromatography of the residue on silica with EtOAc:hexane 30:70 yielded the title compound as an E:Z mixture.

¹H NMR (CD₃COCD₃) 8 5.40 (s, 2H), 5.74 and 6.34 (2d, 1H, E and Z), 7.12-7.61 (m, 6H), 7.72 (d, 1H), 7.98 (d, 1H), 8.01 (s, 1H), 8.39 (d, 1H) p.p.m.

### Step 3 Preparation of methyl 4-(3-(7-chloroquinolin-2-ylmethoxy)phenyl)-5-cyano-3-thiapentanoate

To the a, -unsaturated nitrile (step 2, 756 mg, 2.36 mmoles) in THF (3 mL) at 0°C, methyl thioglycolate (330 µL,1.5 equiv.) and 1,5-diazabicyclo[4.3.0]non-5-ene (145 µL, 0.5 equiv.) were added. After 2 hours of stirring at 0°C, the solvent was evaporated. Flash chromatography of the residue on silica with EtOAc:hexane 30:70 afforded the title compound.

¹H NMR (CDCI₃) 8 2.84-3.03 (m, 2H), 3.00 (d, 1H), 3.14 (d, 1H), 3.70 (s, 3H), 4.35 (dd, 1H), 5.37 (s, 2H), 6.99 (m, 2H), 7.10 (dd, 1 H), 7.30 (dd, 1H), 7.50 (dd, 1 H), 7.67 (d, 1H), 7.77 (dd, 1H), 8.09 (d, 1 H), 8.18 (d, 1 H) p.p.m.

### Step 4 Preparation of N,N-dimethyl 4-(3-(7-chloroquinolin-2-ylmethoxy)phenyl)-5-cyano-3-thiapentanamide

Trimethylaluminum (2.0M in hexane, 4 mL) and dimethylamine (2.0M in toluene, 12 mL) were mixed together at -10°C and stirred at room temperature 30 minutes to give a solution of Me₂AINMe₂ 0.5M. To the cyanoester (step 3,800 mg, 1.87 mmoles) in CH₂CI₂ (4 mL), Me₂AINMe₂ (0.5M, 8 mL, 2 equiv.) was added and the reaction mixture stirred overnight. At 0° C,10% HCI was added dropwise, followed by 25% aqueous NH₄0Ac. Filtration on celite, extraction with EtOAc, evaporation and flash chromatography on silica with EtOAc:toluene 1:1 and EtOAc afforded the title compound.

¹H NMR (CD₃COCD₃) δ 2.84 (s, 3H), 2.97 (s, 3H), 3.12-3.30 (m, 2H), 3.32 (d, 1H), 3.43 (d, 1H), 4.42 (dd, 1H), 5.38 (s, 2H), 7.00-7.36 (m, 4H), 7.59 (dd, 1 H), 7.74 (d, 1H), 7.99 (d, 1H), 8.03 (d, 1 H), 8.39 (d, 1H) p.p.m.

### Step 5

The cyanoamide (step 4,630 mg, 1.43 mmoles) and tributyltin azide (714 mg,1.5 equiv.) were mixed together in hot toluene. The solvent was evaporated and the residual oil heated at 120° C for 4 hours. Dissolution in hot CH₂CI₂ and flash chromatography on silica with acetone:CH₂Cl₂:AcOH 5:95:1, 10:90:1 and 20:80:1 afforded the title compound.

¹H NMR (CD₂CI₂) δ 2.96 (s, 3H), 3.08 (s, 3H), 3.33 (s, 2H), 3.51 (m, 2H), 4.22 (dd, 1H), 5.38 (s, 2H), 6.92-7.01 (m, 2H), 7.08 (broad s, 1H), 7.30 (dd, 1H), 7.53 (dd, 1H), 7.68 (d, 1H), 7.81 (d, 1H), 8.08 (broad s, 1H), 8.23 (d, 1H), p.p.m.; ms 482 (M·+), 409, 396, 233, 177, 176.

### EXAMPLE 8

### N,N-Dimethyl 5-(3-(7-chloroquinolin-2-ylmethoxy)phenyl)-6-(1 H-tetrazol-5-yl)-4-thiahexanamide

Using the procedure of Example 7, but replacing methyl thioglycolate with methyl 3-mercaptopropionate in step 3, the title compound was prepared.

¹H NMR (C0₃COCD₃) δ 2.43-2.75 (m, 4H), 2.89 (s, 3H), 2.94 (s, 3H), 3.50-3.69 (m, 2H), 4.41 (dd, 1H), 5.37 (s, 2H), 6.92-7.01 (m, 2H), 7.16 (broad s, 1 H), 7.25 (dd, 1H), 7.40 (dd, 1H), 7.73 (d, 1H), 8.02 (d, 1 H), 8.05 (broad s, 1H), 8.42 (d, 1H), p.p.m.; ms 496 (M·+), 423, 396, 365, 314, 247, 177, 176.

### EXAMPLE 9

### 5-(3-(7-Chloroquinolin-2-ylmethoxy)phenyl)-6-(1 H-tetrazol-5-yl)-4-thiahexanamide

### Step 1 Preparation of methyl 6-cyano-5-(3-(7-chloroquinolin-2-ylmethoxy)phenyl)-4-thiahexanoate

Using the procedure of Example 7, step -1 to 3, but replacing methyl thioglycolate with methyl 3-mercaptopropionate in step 3, the title compound was prepared.

1H NMR (CDCI₃) δ 2.47 (t, 2H), 2.64 (t, 2H), 2.78-2.98 (m, 2H), 3.69 (s, 3H), 4.12 (dd, 1H), 5.38 (s, 2H), 6.94-7.01 (m, 2H), 7.09 (broad s, 1H), 7.30 (dd, 1H), 7.51 (dd, 1H), 7.69 (d, 1H), 7.78 (d, 1H), 8.09 (broad s, 1H), 8.19 (d, 1H) p.p.m.

### Step 2 Preparation of 5-(3-(7-chloroquinolin-2-ylmethoxy)phenyl)-6-cyano-4-thiahexanamide and 3-(3-(7-chloroquinolin-2-ylmethoxy) phenyl)-4-thiaheptane-1,7-dinitrile

A solution of Me₂AINH₂ 1.3M was prepared by adding an excess of ammonia to a mixture of 9.0 mL of Me₃Al (2.0M in hexane) and 5.0 mL of CH₂CI₂ at -78° C and let it react for 2 hours at room temperature. To the ester (step 1, 700 mg, 1.59 mmoles) in 5 mL of CH₂CI₂, 9.0 mL of Me₂AINH₂ 1.3M were added and the mixture was heated to reflux for 24 hours. Hydrolysis with 10% HCI, followed by addition of 25% NH₄0Ac, extraction with EtOAc drying, evaporation and flash chromatography of the residue on silica with EtOAc:hexane 30:70 and EtOAc afforded the dinitrile and the cyanoamide.

Dinitrile: IR (neat) v 2925, 2250, 1614, 1600, 1498 cm⁻¹; ¹H NMR (CDCI₃) δ 2.40 (t, 2H), 2.62 (t, 2H), 2.88 (d, 2H), 4.22 (t, 1H), 5.39 (s, 2H), 7.00 (m, 2H), 7.10 (broad s, 1 H), 7.32 (dd, 1H), 7.51 (dd, 1H), 7.68 (d, 1H), 7.77 (d, 1H), 8.09 (broad s, 1H), 8.20 (d, 1H) p.p.m.

Cyanoamide: ¹H NMR (CD₃COCD₃) δ 2.32 (t, 2H), 2.61-2.81 (m, 2H), 3.13 (d, 2H), 4.34 (t, 1 H), 5.38 (s, 2H), 6.28 (broad s, 1H), 6.81 (broad s, 1H), 7.00-7.10 (m, 3H), 7.24 (broad s, 1H), 7.31 (dd, 1H), 7.60 (dd, 1H), 7.75 (d, 1H), 8.01 (d, 1H), 8.04 (broad s, 1H), 8.41 (d, 1H) p.p.m.

### Step 3

Starting from the cyanoamide (step 2) and using the procedure of Example 7, step 5, the title compound was obtained. It was recrystallized from THF:ether.

¹H NMR (CD₃COCD₃) 8 2.35 (t, 2H), 2.45-2.72 (m, 2H), 3.47-3.56 (m, 2H), 4.50 (dd, 1 H), 5.36 (s, 2H), 6.47 (broad s, 1 H), 6.89-7.01 (m, 3H), 7.10 (broad s, 1H), 7.17 (broad s, 1 H), 7.22 (dd, 1 H), 7.60 (dd, 1H), 7.72 (d, 1H), 8.02 (d, 1H), 8.05 (broad s, 1H), 8.41 (d, 1H), p.p.m.; ms 468 (M·+), 397, 314, 176, 175.

### EXAMPLE 10

### 5,5'-(2-(3-(7-Chloroquinolin-2-ylmethoxy)phenyl)-3-thiapentane-1,5-diyl)bis 1H-tetrazole

Using the procedure of Example 7, step 5, but adding 3 equiv. of tributyltin azide instead of 1.5 equiv., the dinitrile obtained in Example 9, step 2, was converted to the title compound.

¹H NMR (CD₃COCD₃) δ 2.82 (t, 2H), 3.18 (t, 2H), 3.60 (d, 2H), 4.54 (t, 1H), 5.38 (s, 2H), 6.92-7.02 (m, 2H), 7.19 (broad s, 1H), 7.26 (dd, 1H), 7.60 (dd, 1 H), 7.74 (d, 1H), 8.02 (d, 1H), 8.04 (broad s, 1H), 8.41 (d, 1H), p.p.m.

### EXAMPLE 11

### Methyl N-(5-(3-(7-chloroquinolin-2-ylmethoxy)phenyl)-6-(iH-tetrazol-5-yl)-4-thiahexanoyl) glycinate

### Step 1 Preparation of 5-(3-(7-chloroquinolin-2-ylmethoxy)phenyl)-6-cyano-4-thiahexanoic acid

To the propenenitrile (Example 7, step 2, 1.075 g, 3.35 mmoles) in 3 mL of THF, 3-mercaptopropionic acid (440 µL, 1.5 equiv.) and 1,5-diazabicyclo[4.3.0]non-5-ene (830 µL, 2.0 equiv.) were added and the reaction mixture stirred 20 hours. Addition of 25% NH₄0Ac and AcOH, extraction with THF:EtOAc 1:1, drying, evaporation and flash chromatography of the residue on silica using EtOAc:toluene:AcOH 25:75:1 afforded the title compound.

¹H NMR (CD₃COCD₃) δ 2.52 (t, 2H), 2.60-2.80 (m, 2H), 3.14 (d, 2H), 4.37 (t, 1 H), 5.39 (s, 2H), 7.00-7.15 (m, 2H), 7.27 (broad s, 1H). 7.32 (dd, 1H), 7.60 (dd, 1 H), 7.75 (d, 1H). 8.00 (d, 1 H), 8.05 (broad s, 1H), 8.40 (d, 1H), p.p.m. Step 2 Preparation of methyl N-(5-(3-(7-chloroquinolin-2-ylmethoxy)phenyl)-6-cyano-4-thiahexanoyl)glycinate

At 0°C, dimethylformamide (50 µL) and oxalyl chloride (440 µL, 1.5 equiv.) were added to a solution of the cyanoacid (step 1, 1.40 g, 3.28 mmoles) in 15 mL of CH₂CI₂. After 20 minutes of stirring at room temperature, the reaction mixture was cooled to 0°C, methyl glycinate (1.136g, ~ equiv.) added and the mixture stirred for another 30 minutes at room temperature. Addition of 25% NH₄0Ac, extraction with EtOAc, drying, evaporation and flash chromatography on silica with EtOAc:toluene 50:50 and 70:30 afforded the title compound.

1H NMR (CD₃COCD₃) 8 2.50 (t, 2H), 2.62-2.84 (m, 2H), 3.12 (d, 2H), 3.65 (s, 3H), 3.95 (d, 2H), 4.36 (t, 1 H), 5.39 (s, 2H), 7.02 (dd, 1H), 7.09 (d, 1H), 7.25 (broad s, 1H), 7.31 (dd, 1H), 7.53 (broad s, 1H), 7.60 (dd, 1H), 7.75 (d, 1H), 8.00 (d, 1H), 8.05 (broad s, 1H), 8.40 (d, 1H) p.p.m.

### Step 3

Using the procedure in Example 7, step 5, the compound of step 2 converted to the title compound.

1H NMR (CD₃COCD₃) 8 2.48 (t, 2H), 2.53-2.77 (m, 2H), 3.48-3.68 (m, 2H), 3.66 (s, 3H), 3.97 (m, 2H), 4.45 (t, 1H), 5.36 (s, 2H), 6.95 (dd, 1 H), 6.99 (d, 1 H), 7.18 (broad s, 1H), 7.24 (dd, 1 H), 7.60 (dd, 1 H), 7.73 (d, 1 H), 8.02 (d, H), 8.04 (broad s, 1H), 8.41 (d, 1H), p.p.m.; ms 410 (M -CH₂CONHCH₂COOMe), 177.

### EXAMPLE 12

### N-(5-(3-(7-Chloroquinolin-2-ylmethoxy)phenyl)-6-(1H-tetrazol-5-yl)-4-thiahexanoyl) glycine

The title compound of Example 11, (208 mg, 384 µmoles) was hydrolyzed with 80 µL NaOH 10N in 6 mL MeOH:THF:H₂0 1:1:1 during an hour. AcOH and 25% NH₄0Ac were added, the organic solvents evaporated and the remaining mixture extracted with EtOAc:THF 1:1. The solvents were evaporated, the product dissolved in MeOH and filtered on celite. Evaporation of solvent gave a solid which was swished with ether to afford the pure title compound.

¹H NMR (CD₃COCD₃:DMSO) 8 2.40 (t, 2H), 2.50-2.67 (m, 2H), 3.50 (d, 2H), 3.80 (d, 2H), 4.49 (t, 1H), 5.38 (s, 2H), 6.94 (dd, 1H), 6.99 (d, 1H), 7.18 (broad s, 1H), 7.24 (dd, 1H), 7.63 (dd, 1H), 7.75 (d, 1H), 8.02-8.10 (m, 2H), 8.47 (d. 1H) p.p.m.; ms 526 (M·+), 423, 397, 365, 314, 177.

### EXAMPLE 13

### 6-(3-(7-Chloroquinolin-2-ylmethoxy)phenyl)-7-(1 H-tetrazol-5-yl)-5-thiaheptanamide

### Step 1 Preparation of 6-(3-(7-chloroquinolin-2-ylmethoxy)phenyl)-7-cyano-5-thiaheptanoic acid

Using the procedure of Example 11, step 1, but replacing 3-mercaptopropionic acid with 4-mercaptobuta- noic acid, the title compound was prepared.

¹H NMR (CD₃COCD₃) 8 1.79 (m, 2H), 2.34 (t, 2H), 2.51 (t, 2H), 3.12 (d, 2H), 4.29 (t, 1H), 5.39 (s, 2H), 7.03 (dd, 1H), 7.12 (d, 1H), 7.25 (broad s, 1H), 7.32 (dd, 1H), 7.60 (dd, 1H), 7.75 (d, 1H), 8.01 (d, 1H), 8.06 (broad s, 1H), 8.41 (d, 1H), p.p.m.

### Step 2 Preparation of 6-(3-(7-chloroquinolin-2-ylmethoxy)phenyl)-7-cyano-5-thiaheptanamide

Using the procedure of Example 11, step 2, but replacing methyl glycinate with an excess of ammonia, the title amide was prepared.

¹H NMR (CD₃COCD₃) 1.79 (m, 2H), 2.22 (t, 2H), 2.49 (t, 2H), 3.12 (d, 2H), 4.30 (t, 1H), 5.39 (s, 2H), 6.12 (broad s, 1H), 6.71 (broad s, 1H), 7.03 (dd, 1H), 7.10 (d, 1H), 7.23 (broad s, 1H), 7.31 (dd, 1H), 7.60 (dd, 1H), 7.76 (d,1H), 8.02 (d, 1H), 8.03 (broad s, 1H), 8.42 (d, 1H) p.p.m.

### Step 3

Using the procedure in Example 7, step 5, the amidonitrile (step 2) was converted to the title compound. ¹H NMR (CD₃COCD₃:DMSO) δ 1.72 (m, 2H), 2.23 (m, 2H), 2.36 (t, 2H), 3.50 (d, 2H), 4.43 (t, 1H), 5.38 (s, 2H), 6.53 (broad s, 1 H), 6.94 (dd, 1H), 6.96 (d, 1 H), 7.10 (broad s, 1 H), 7.15 (broad s, 1 H), 7.23 (dd, 1H), 7.63 (dd, 1H), 7.75 (d, 1H), 8.05 (d, 1H), 8.07 (broad s, 1H), 8.44 (d, 1H) p.p.m.; ms 437 (M -CONH₂ - H), 390, 365, 177.

### EXAMPLE 14

### 5,5'-(2-(3-(7-Chloroquinolin-2-ylmethoxy)phenyl)-3-thiahexane-1,6-diyl)bis 1H-tetrazole

### Step 1 Preparation of 3-(3-(7-chloroquinolin-2-ylmethoxy)phenyl)-4-thiaoctane-1,8-dinitrile

To the cyanoamide (Example 13, step 2, 407 mg, 925 µmoles) in 10 mL of anhydrous THF at -10°C, pyridine (450 µL, 6 equiv.) and trifluoroacetic anhydride (143 µL, 1.1 equiv.) were added and the reaction mixture stirred 20 minutes at -10°C. Addition of 25% NH₄0Ac, extraction with EtOAc, drying, evaporation and flash chromatography of the residue on silica with EtOAc:toluene 10:90 and 15:85 afforded the title compound. ¹H NMR (CD₃COCD₃) δ 1.83 (m, 2H), 2.50 (t, 2H), 2.59 (t, 2H), 3.13 (d, 2H), 4.35 (t, 1H), 5.39 (s, 2H), 7.04 (dd, 1H), 7.10 (d, 1H), 7.26 (broad s, 1H), 7.33 (dd, 1H), 7.60 (dd, 1H), 7.74 (d, 1H), 8.00 (d, 1H), 8.03 (broad s, 1H), 8.40 (d, 1H) p.p.m.

### Step 2

Using the procedure of Example 7, step 5, but adding 3.0 equiv. of tributyltin azide instead of 1.5 equiv., the dinitrile from step 1 was converted to the title compound.

¹H NMR (CD₃COCD₃) 8 1.95 (m, 2H), 2.46 (t, 2H), 2.97 (m, 2H), 3.58 (d, 2H), 4.45 (t, 1H), 5.37 (s, 2H), 6.90-7.00 (m, 2H), 7.14 (broad s, 1H), 7.22 (dd, 1H), 7.60 (dd, 1H), 7.73 (dd, 1H), 8.01 (d, 1H), 8.03 (broad s, 1H), 8.42 (d, 1 H) p.p.m.

### EXAMPLE 15

### 7-(N-t-Butylcarbamyl)-4-(3-(7-chloroquinolin-2-ylmethoxy)phenyl)-3-methyl-5-thiaheptanoic acid

### Step 1 Preparation of 1-(3-methoxyphenyl)propanol

To 10.0 mL of m-anisaldehyde in 150 mL ether at -78° C, 33 mL of EtMgBr 3.0M in ether were added dropwise. After 30 minutes at 0°C, aqueous NH₄CI and 25% NH₄0Ac were added. Extraction with EtOAc afforded the title compound, which was used as such for the next step.

### Step 2 Preparation of 1-(3-methoxyphenyl)propan-1-one

To the crude product of step 1 in 500 mL of CH₂CI₂, 145 g of milled molecular sieves (4 ©) and pyridinium chlorochromate (60 g, 3 equiv.) were added. The reaction mixture was stirred for an hour and filtered through silica gel. The silica was then washed with CH₂CI₂ and EtOAc:hexane 20:80. The filtrate was evaporated to afford the desired ketone as a yellowish oil.

### Step 3 Preparation of methyl 4-(3-methoxyphenyl)-3-methyl-4-oxobutanoate

To potassium hexamethyldisilazane 0.60M in toluene (150 mL, 1.1 equiv) were added 150 mL of anhydrous THF. The reaction was cooled to -78°C and a solution of the ketone from step 2 in THF (120 mL), was added dropwise. After one hour of stirring at -78°C, methyl bromoacetate (9.4 mL, 1.2 equiv.) was added and the mixture was stirred 15 minutes at -78°C and 15 min at room temperature. The reaction was quenched at 0°C with 25% NH₄0Ac. Extraction with EtOAc and flash chromatography on silica with EtOAc:hexane 10:90 and 20:80 afforded the title ketoester.

¹H NMR (CD₃COCD₃) 8 1.18 (d, 3H), 2.48 (dd, 1H), 2.87 (dd, 1H), 3.59 (s, 3H), 3.86 (s, 3H), 3.97 (m, 1H), 7.20 (dd, 1H), 7.45 (dd, 1H), 7.50 (m, 1H), 7.62 (broad d, 1H) p.p.m.

### Step 4 Preparation of 4-(3-methoxyphenyl)-3-methyl-4-oxobutanoic acid

To the ketoester (step 3,14.414 g, 61 mmoles),150 mL MeOH, 75 mLTHF, 60 mL H₂0 and 30 mL NaOH 10N were added and the solution stirred 30 minutes. Then, 25% NH₄0Ac and 30 mL conc. HCL were added and the organic solvents evaporated. Extraction of the remaining aqueous layer with EtOAc and evaporation afforded the acid as a white solid.

¹H NMR (CD₃COCD₃) δ 1.18 (d, 3H), 2.46 (dd, 1H), 2.89 (dd, 1H), 3.85 (s, 3H), 3.95 (m, 1H), 7.20 (dd, 1H), 7.45 (dd, 1H), 7.50 (m, 1H), 7.63 (broad d, 1H) p.p.m.

### Step 5 Preparation of cis 5-(3-methoxyphenyl)-4-methyldihydrofuran-2-(3H)-one

To the ketoacid (step 4, 9.00 g, 40.5 mmoles) in 200 mL THF, ZnCl₂ 1.OM in THF (43 mL, 1.05 equiv.) was added. Then, at -78° C, diisobutylaluminum hydride 1.5M in toluene (65 mL, 2.4 equiv.) was added dropwise and the suspension stirred mechanically at -78° C for 3:30 hours. Hydrolysis at -78° C with 10% HCl, extraction with EtOAc, drying and evaporation afforded a mixture of the hydroxyacid and the lactone. The hydroxyacid:lactone mixture was dissolved in 70 mL of CH₂CI₂ and trifluoroacetic acid (140 µL) was added. The solution was stirred at room temperature 8 hours and left at 5° C until lactonization was completed (~70 hours). Then, toluene was added and the solvents evaporated. The lactone was finally purified by flash chromatography on silica with EtOAc:toluene 5:95.

¹H NMR (CDCI₃) 8 0.73 (d, 3H), 2.37 (d, 1H), 2.82 (dd, 1H), 2.88 (m, 1H), 3.83 (s, 3H), 5.59 (d, 1H), 6.80-6.90 (m, 3H), 7.30 (dd, 1H) p.p.m.

### Step 6 Preparation of 7-(N-t-butylcarbamyl)-4-(3-methoxyphenyl)-3-methyl-5-thiaheptanoic acid

To the lactone (step 5, 456 mg, 2.21 mmoles) in 11 mL of CH₂CI₂ at -10°C, N-t-butyl 3-mercaptopropanamide (537 mg, 1.5 equiv.) and AlCl₃ (1.473 g, 5 equiv.) were added. After 1.5 hours of stirring at 0°C, 25% NH₄0Ac and AcOH were added. Extraction with EtOAc, drying, evaporation and flash chromatography of the residue on silica with EtOAc:toluene:AcOH 25:75:1, 40:60:1 and 60:40:1 afforded the starting material, the title compound and 7-(N-t-butylcarbamyl)-4-(3-hydroxyphenyl)-3-methyl-5-thiaheptanoic acid (for data, see next step).

Title compound (two diastereomers): ¹H NMR (CD₃COCD₃) δ 0.87 and 1.09 (2d, 3H), 1.30 and 1.33 (2s, 9H), 2.08-2.55 (m, 6H), 2.81 and 2.91 (2ddm, 1H), 3.81 (s, 3H), 3.83 and 3.90 (2d, 1 H), 6.69 (broad s, 1H), 6.82 (broad d, 1H), 6.90-6.98 (m, 2H), 7.25 (broad dd, 1H) p.p.m.

### Step 7 Preparation of 7-(N-t-butylcarbamyl)-4-(3-hydroxyphenyl)-3-methyl-5-thiaheptanoic acid

To the title compound of step 6 (540 mg, 1.47 mmoles) in 7 mL CH₂CI₂ at -78°C, triethylamine (225 µL, 1.1 equiv.) and BBr₃ (1.0M in CH₂CI₂, 6 mL, 4 equiv.) were added. After one hour of stirring at room temperature, 25% NH₄0Ac was added. Extraction with EtOAc, drying, evaporation and flash chromatography on silica using EtOAc:toluene:AcOH 40:60:1 and 60:40:1 yielded the title compound (2 diastereomers).

¹H NMR (CD₃COCD₃) 8 0.88 and 1.06 (2d, 3H), 2.29 and 2.32 (2s, 9H), 2.10-2.60 (m, 6H), 2.81 and 2.92 (2dd, 1H), 3.78 and 3.85 (2d, 1H), 6.73 (broad d, 1H), 6.79-6.91 (m, 1H), 6.97 (broad s, 1H), 7.10-7.28 (m, 2H) p.p.m.

### Step 8 Preparation of methyl 7-(N-t-butylcarbamyl)-4-(3-hydroxyphenyl)-3-methyl-5-thiaheptanoate

An excess of diazomethane in ether was added at 0° C to the acid (step 7, 420 mg) in 6 mL THF. The mixture was stirred 30 minutes at 0°C, the solvents evaporated and the residue was purified by chromatography on silica using EtOAc:toluene 30:70 and 40:60.

¹H NMR (CD₃COCD₃) 8 0.82 and 1.03 (2d, 3H, 2 diastereomers), 1.29 (s, 9H), 2.05-2.55 and 2.68 (m + dd, 7H), 3.58 and 3.62 (2s, 3H), 3.75 and 3.82 (2d, 1 H), 6.68-6.90 (m, 4H), 7.13 (dd, 1 H), 8.44 (s, 1 H) p.p.m.

### Step 9 Preparation of methyl

### 7-(N-t-butylcarbamyl)-4-(3-(7-chloroquinolin-2-ylmethoxy)phenyl)-3-methyl-5-thiaheptanoate

A mixture of the phenol (step 8, 285 mg, 776 µmoles), 2-bromomethyl 7-chloroquinoline from preparation 1 (272 mg, 1.4 equiv.), milled K₂CO₃ (208 mg, 2 equiv.) and 5 mL of acetone was heated to reflux for 7.5 hours. Then, EtOAc was added, the mixture was filtered through celite and evaporated. The residue was purified by flash chromatography on silica using EtOAc:toluene 25:75 to afford the title compound.

### Step 10

The ester (step 9, 399 mg, 735 µmoles), 1,2-dimethoxyethane (10 mL) and 1.0N LiOH (1.5 mL, 2 equiv.) were stirred at room temperature for 45 hours. Water and 10% HCI were added, the product was extracted with EtOAc, dried, evaporated and purified by flahs chromatography on silica with EtOAc:toluene:AcOH 40:60:1. ¹H NMR (CD₃COCD₃) δ 0.81 and 1.04 (2d, 3H, 2 diastereomers), 1.29 (s, 9H), 2.10-2.55 and 2.79 (m + dd, 7H), 3.85 and 3.93 (2d, 1H), 5.40 (s, 2H), 6.68 (broad s, 1H), 6.94-7.03 (m, 2H), 7.14 (broad d, 1H), 7.28 (broad dd, 1H), 7.60 (dd, 1H), 7.76 (d, 1H), 8.02 (d, 1H), 8.05 (broad s, 1H), 8.42 (d, 1H) p.p.m.; ms 528 (M·+), 469 (M - C₂H₃0₂), 400 (M - C₇H₁₄NO), 369, 218, 177, 176.

### EXAMPLE 16

### 5-(3-(7-Chloroquinolin-2-ylmethoxy)phenyl)-8-methyl-4-thiadecanedioic acid

### Step 1 Preparation of methyl 3-methyl 5-(methylsulfonyloxy)pentanoate

To methyl 5-hydroxy-3-methylpentanoate (B. Lythose, J. Chem. Soc., Perkin Trans. I, 834 (1978)) (9.63 g, 65.9 mmoles) in 200 mL CH₂CI₂ at -78° C, Et₃N (14 mL, 1.5 equiv.) and methanesulfonyl chloride (5.6 mL, 1.1 equiv.) were added. After one hour of stirring at room temperature, 25% NH₄0Ac was added. Extraction with CH₂CI₂, filtration through silica and evaporation afforded the title compound, which was used as such in the next step.

### Step 2 Preparation of methyl 5-iodo-3-methylpentanoate

The mesylate (step 1, 14.4 g, 64.2 mmoles) and Nal (48 g, 5 equiv.) were heated to reflux in 200 mL acetone for 3 hours. The mixture was then filtered through celite and the solvent evaporated. The residue was partionned between water and Et₂0, the ether extract washed with 50/0 Na₂S₂0₃ and brine, dried and evaporated. Flash chromatography of the residue on silica with EtOAc:hexane 2.5:97.5 afforded the title compound.

¹H NMR (90 MHz, CDCI₃) δ 1.00 (d, 3H), 1.70-2.43 (m, 5H), 3.20 (t, 2H), 3.67 (s, 3H) p.p.m.

### Step 3 Preparation of (5-methoxy-3-methyl-5-oxo-pentyl)triphenyl-phosphonium iodide

Triphenylphosphine (14.6 g, 2 equiv.) and the iodide (step 2,7.850 g, 27.6 mmoles) were heated to 80°C in 50 mL toluene for 24 hours and at 100°C 6 hours. The mixture was allowed to cool to room temperature, the toluene layer was discarded and the remaining oil heated in toluene for another hour. At room temperature, the toluene layer was removed. The oil was heated for one hour in ether, the ether was removed at room temperature and the oil dried under vacuum.

¹H NMR (CDCI₃) 8 1.09 (d, 3H), 1.50-2.00 (m, 3H), 2.26-2.45 (m, 2H), 3.59 (s, 3H), 3.50-3.85 (m, 2H), 7.69-7.90 (m, 15H) p.p.m.

### Step 4 Preparation of methyl 6-(3-methoxyphenyl)-3-methyl-5-hexenoate

The phosphonium salt (step 3, 2.953 g, 5.70 mmoles) was dissolved in 30 mL of hot THF and 1.5 mL of HMPA. At -78° C and under a continuous flow of N₂, potassium hexamethyldisilazane (0.67M in toluene, 7.8 mL, 5.23 mmoles) was added dropwise and the yellow suspension was stirred 50 minutes at this temperature. m-Anisaldehyde (450 µL, 3.70 mmoles) was then added and the green solution was stirred 40 min at -78°C and 1 hour at room temperature. 25% NH₄0Ac was added and the aqueous layer was extracted with EtOAc.

The organic layer was washed two times with water, dried and evaporated. The residue was purified by flash chromatography on silica with EtOAc:hexane 5:95 to afford the title compound.

¹H NMR (CDCI₃) 80.98 (d, 3H), 2.05-2.43 (m, 5H), 2.67 (s, 3H), 2.83 (s, 3H), 5.66 (td, 1H), 6.48 (d, 1H), 6.75-6.88 (m, 3H), 7.27 (dd, 1H) p.p.m.

### Step 5 Preparation of dimethyl 5-(3-methoxyphenyl)-8-methyl-4-thiadecanedioate

To methyl 3-mercaptopropionate (750 µL, 2 equiv.) and the styrene (step 4, 835 mg, 3.36 mmoles) in 17 mL cyclohexane, titanium tetrachloride (1.9 mL, 5 equiv.) was added dropwise and the mixture stirred mechanically 4.25 hours. Then, 25% NH₄0Ac was slowly added at 0°C. Extraction with EtOAc, drying, evaporation and flash chromatography of the residue on silica with EtOAc:toluene 2.5:97.5 afforded the title compound.

¹H NMR (CDCI₃) 8 0.89 and 0.92 (2d, 3H, 2 diastereomers), 1.02-1.48 (m, 2H), 1.75-2.98 (m, 9H), 3.54 (s, 3H), 3.57 (s, 3H), 3.69 and 3.72 (2dd, 1H), 3.82 (s, 3H), 6.68 (dd, 1H), 6.85 (s, 1H), 6.87 (d, 1H), 7.24 (dd, 1H) p.p.m.

### Step 6 Preparation of dimethyl 5-(3-hydroxyphenyl)-8-methyl-4-thiadecanedioate

At -78° C, 9.0 mL BBr₃ 1.OM in CH₂CI₂ were added dropwise to a solution of the diester (step 5, 838 mg, 2.27 mmoles) in 9 mL CH₂Cl₂. The solution was then stirred 2 hours at -10°C. Hydrolysis at -10°C with 25% NH₄0Ac, extraction with EtOAc, drying, evaporation and flash chromatography of the residue on silica with EtOAc:toluene 15:85 afforded the title compound.

IR (neat) v 3405, 2950, 2925, 1735, 1600, 1590, 1453, 1437 cm -¹; ¹H NMR (CDCI₃) 8 0.91 (2d, 3H, 2 diastereomers), 1.03-1.45 (m, 3H), 1.75-2.32 (m, 4H), 2.46 (m, 2H), 2.56 (m, 2H), 3.65 (s, 3H), 3.68 (s, 3H), 3.70 (dd, 1H), 5.60 (2 broad s, 1H), 6.73 (dd, 1H), 6.82 (s, 1H), 6.83 (d, 1H), 7.19 (dd, 1H) p.p.m.

### Step 7 Preparation of dimethyl 5-(3-(7-chloroquinolin-2-ylmethoxy)phenyl)-8-methyl-4-thiadecanedioate

Using the experimental procedure of Example 15, step 9, the phenol (step 6) was converted to the title compound.

¹H NMR (CD₃COCD₃) 8 0.83 (2d, 3H, 2 diastereomers), 0.98-1.45 (m, 3H), 1.72-1.92 (m, 3H), 2.15-2.28 (m, 1H), 2.35-2.55 (m, 4H), 3.57 (s, 3H), 3.60 (s, 3H), 3.84 (dd, 1 H), 5.38 (s, 2H), 6.96 (m, 2H), 7.11 (broad s, 1H), 7.26 (dd, 1H), 7.59 (dd, 1H), 7.74 (d, 1H), 8.00 (d, 1H), 8.04 (broad s, 1H), 8.40 (d, 1H) p.p.m.

### Step 8

Using the procedure of Example 15, step 10, but adding 3.0 equiv. of LiOH to the diester (step 7) instead of 2.0, the title diacid was obtained.

¹H NMR (CD₃COCD₃) δ 0.88 (2d, 3H, 2 diastereomers), 0.97-1.50 (m, 3H), 1.75-1.93 (m, 3H), 2.13-2.29 (m, 1H), 2.37-2.54 (m, 4H), 3.87 (t, 1H), 5.40 (s, 2H), 6.92-7.02 (m, 2H), 7.13 (broad s, 1H), 7.25 (dd, 1H), 7.60 (dd, 1H), 7.74 (d, 1H), 8.02 (d, 1H), 8.05 (broad s, 1H), 8.42 (d, 1H) p.p.m.; ms 501 (M·+) 483 (M-H₂O), 450, 429 (M - CH₂=CH₂-C0₂), 396 (M - SCH₂CH₂C0₂H), 179, 178, 177, 176, 141.

### EXAMPLE 17

### 9-(N-t-Butylcarbamyl)-6-(3-(7-chloroquinolin-2-ylmethoxy)phenyl)-3-methyl-7-thianonanoic acid

### Step 1 Preparation of methyl

### 9-(N-t-butylcarbamyl)-6-(3-(7-chloroquinolin-2-ylmethoxy)phenyl)-3-methyl-7-thianonanoate

At -10°C, AlCl₃ (581 mg, 10 equiv.) was added to a solution of the diester (Example 16, Step 7, 230 mg, 434 µmoles) and N-t-butyl 3-mercaptopropanamide (282 mg, 4 equiv.) in 2 mL CH₂CI₂. After 3.3 hours of stirring at 0°C, 25% NH₄0Ac was added. Extraction with EtOAc, drying, evaporation and flash chromatography of the residue on silica with EtOAc:hexane 25:75 and 30:70 afforded the desired compound, contaminatec with a little quantity of the disulfide of methyl 3-mercaptopropionate. The title compound was finally purified b y HPLC (µ Porasil column) using EtOAc:hexane 25:75 as eluant.

¹H NMR (CD₃COCD₃) δ 0.83 (2d, 3H, 2 diastereomers), 0.95-1.42 (m, 3H), 1.30 (s, 9H), 1.72-1.90 (m, 3H), 1.95-2.09 and 2.13-2.28 (m, 3H), 2.49 (t, 2H), 3.58 (s, 3H), 3.72 (t, 1H), 5.38 (s, 2H), 6.68 (broad s, 1H), 6.94 (broad d, 2H), 7.10 (broad s, 1H), 7.25 (dd, 1H), 7.59 (dd, 1H), 7.73 (d, 1H), 7.98 (d, 1H), 8.04 (broad s, 1H), 8.39 (d, 1H) p.p.m.

### Step 2

Using the procedure of Example 15, step 10, the ester was hydrolysed to the title acid.

¹H NMR (CD₃COCD₃) δ 0.86 (2d, 3H, 2 diastereomers), 1.00-1.50 (m, 3H), 1.29 (s, 9H), 1.75-1.85 (m, 3H), 2.13-2.30 (m, 3H), 2.49 (broad t, 2H), 3.83 (t, 1H), 5.39 (s, 2H), 6.70 (broad s, 1H), 6.92-7.00 (m, 2H), 7.10 (broad s, 1H), 7.25 (dd, 1H), 7.60 (dd, 1H), 7.75 (d, 1H), 8.01 (d, 1H), 8.04 (broad s, 1H), 8.41 (d, 1H) p.p.m.; ms 556 (M·+), 428 (M - C₇H₁₄NO), 397 (M - C₇H₁₃NOS), 296, 177.

### EXAMPLE 18

### 5-(3-(7-Chloroquinolin-2-ylmethoxy)phenyl)-9-(N,N-dimethylcarbamyl-4-thianonanoic acid

### Step 1 Preparation of N,N-dimethyl 6-(3-methoxyphenyl)-3-methyl-5-hexenamide

Using the procedure of Example 7, step 4, the styrene-ester from Example 16, step 4 was converted to the title compound.

### Step 2 Preparation of methyl 9₋(N,N-dimethylcarbamyl)-5-(3-methoxyphenyl)-8-methyl-4-thianonanoate

TiCl₄(1.8 mL, 8 equiv.) was added dropwise to a stirred solution of the amide (step 1, 540 mg, 2.07 mmoles) and methyl 3-mercaptopropionate (920 µL, 4 equiv.) in 15 mL cyclohexane. An oil separated and the mixture was stirred 4 days in the dark. Then, at 0°C, 25% NH₄0Ac was added. Extraction with EtOAc, drying, evaporation and flash chromatography of the residue on silica with EtOAc:toluene 40:60, 60:40 and 80:20 afforded the title compound.

¹H NMR (CDCI₃) δ 0.90 (2d, 3H, 2 diastereomers), 1.00-1.50 (m, 2H), 1.75-2.30 (m, 5H), 2.45 (m, 2H), 2.55 (m, 2H), 2.93 (s,3H), 2.97 (s,3H),3.67 (s,3H),3.73 (t,1H),3.82 (s,3H),6.78 (dd, 1 H), 6.86 (s,1H),6.87 (d,1H), 7.23 (dd, 1H) p.p.m.

### Step 3

Using the procedures of Example 16, step 6 and Example 15, step 9 and 10, the product of step 2 was converted to the title compound.

¹H (CD₃COCD₃) 8 0.83 (2d, 3H, 2 diasteromers), 0.93-1.48 (m, 2H), 1.71-2.28 (m, 5H), 2.42 (m, 2H), 2.49 (m, 2H), 2.84 (2s, 3H), 2.98 (2s, 3H), 3.87 (t, 1H), 5.39 (s, 2H), 6.92-7.00 (m, 2H), 7.13 (broad s, 1H), 7.26 (dd, 1H), 7.60 (dd, 1H), 7.75 (d, 1H), 8.01 (d, 1H), 8.04 (broad s, 1H), 8.40 (d, 1H) p.p.m.; exact mass calcd. for C₂₈H₃₄CIN₂0₄S (M + 1) 529.1929, found 529.1929.

### EXAMPLE 19

### N,N-Dimethyl 5-(3-(7-chloroquinolin-2-ylmethoxy)phenyl)-8-(1H-tetrazol-5-yl)-4-thiaoctanamide

### Step 1 Preparation of methyl 5-hydroxy-5-(3-methoxyphenyl)pentanoate

A solution of 3-methoxyphenyl magnesium bromide was prepared with 3-bromoanisole (5.748 g, 30.7 mmoles) and magnesium (0.845 g, 34.8 mmoles) in 30 mL THF. To methyl 5-oxopentanoate (prepared by the method of A.W. Burgstahler, Synthesis 1976,767) (4.015 g, 30.85 mmoles) in 20 mL THF at -78° C, the Grignard reagent was added slowly. The reaction mixture was stirred 10 minutes at -78°C and 40 minutes at room temperature. Hydrolysis with 25% NH₄0Ac at 0°C, extraction with EtOAc, drying, evaporation and flash chromatography on silica with EtOAc:hexane 35:65 afforded the title compound, contaminated with the corresponding lactone and 3,3'-(1-oxo-5-hydroxypentane-1,5-diyl)bismethoxybenzene. The mixture was used as such in the next step.

### Step 2 Preparation of 6-(3-methoxyphenyl)tetrahydropyran-2-one

To the impure hydroxyester of step 1, 14 mLTHF, 23 mL MeOH, 9 mL H₂0 and 10 mL NaOH 10N were added. The solution was stirred 3 hours and then water and conc. HCI were added until pH 9. The aqueous layer was washed with EtOAc and acidified to pH 3. Extraction with EtOAc and evaporation of solvents afforded the hydroxy acid as an oil. The oil was dissolved in CH₂CI₂ and 10 µL trifluoracetic acid added. The solution was stirred 3 hours at room temperature and 2.5 days at 5°C. Solvents were evaporated and the residue was purified by flash chromatography on silica with EtOAc:hexane 40:60 to afford the title compound.

¹H NMR (CDCI₃) 8 1.80-2.05 (m, 3H), 2.12-2.23 (m, 1H), 1.50-1.80 (m, 2H), 3.83 (s, 3H), 5.35 (dd, 1H), 6.83-6.96 (m, 3H), 7.30 (dd, 1H) p.p.m.

### Step 3 Preparation of 8-(N,N-dimethylcarbamyl)-5-(3-hydroxyphenyl)-6-thiaoctanoic acid

At -10°C, AlCl₃ (2,146 g, 6 equiv.) was added to a solution of the lactone (step 2, 555 mg, 2.69 mmoles) and N,N-dimethyl 3-mercaptopropanamide (1.437 g, 4 equiv.) in 14 mL CH₂Cl₂. A viscous oil separated and stirring was continued for 2 hours at 0°C. 250/o NH₄0Ac and AcOH were added and the aqueous layer extracted with EtOAc, dried and evaporated. Flash chromatography of the residue on silica with EtOAc:toluene:AcOH 20:80:1, 30:70:1 and 40:60:1 afforded 8-(N,N-dimethylcarbamyl)-5-(3-methoxyphenyl)-6-thianonanoic acid and the title compound. The methyl ether of the first product was further cleaved, using the procedure of Example 15, step 7, to afford the title compound.

¹H NMR (CDCI₃) δ 1.55-1.73 (m, 2H), 1.88 (m, 2H), 2.28-2.47 (m, 4H), 2.64 (t, 2H), 2.89 (s, 3H), 2.92 (s, 3H), 3.74 (t, 1H), 6.73 (d, 1H), 6.80-6.90 (m, 2H), 7.15 (dd, 1H) p.p.m.

### Step 4 Preparation of 7-chloroquinolin-2-ylmethyl

### 5-(3-(7-chloroquinolin-2-ylmethoxy)phenyl)-8-(N,N-dimethylcarbamyl-6-thiaoctanoate

Using the procedure of Example 15, step 9, but doubling the amount of reactants, the phenol (step 3) was converted to the title compound.

¹H NMR (CDCI₃) δ 1.60-2.00 (m, 4H), 2.32-2.46 (m, 4H), 2.52-2.69 (m, 2H), 2.86 (s, 3H), 2.90 (s, 3H), 3.77 (dd, 1H), 5.34 (s, 2H), 5.36 (s, 2H), 6.83-6.94 (m, 2H), 7.02 (broad s, 1H), 7.23 (dd, 1H), 7.42 (d, 1H), 7.45-7.52 (m, 2H), 7.65-7.79 (m, 3H), 8.07 (broad s, 2H), 8.11-8.20 (m, 2H) p.p.m.

### Step 5

The ester function of the product of step 4 was converted to the tetrazole in four steps. First, the ester was hydrolyzed to the acid using the procedure of Example 15, step 10, but the reaction time was shortened to 16 hours. Second, the acid function was converted to the amide using the procedure of Example 11, step 2, but replacing methyl glycinate by an excess of ammonia. Third, the nitrile was formed using the procedure of Example 14, step 1. Finally, the tetrazole (title compound) was obtained using the procedure of Example 7, step 5.

¹H NMR (CD₃COCD₃) 8 1.63-1.95 (m, 4H), 2.35-2.54 (m, 4H), 2.83 (s, 3H), 2.90 (s, 3H), 2.92 (t, 2H), 3.95 (t, 1H), 5.38 (s, 2H), 6.92-7.00 (m, 2H), 7.12 (broad s, 1H), 7.25 (dd, 1H), 7.60 (dd, 1H), 7.74 (d, 1H), 8.01 (d, 1H), 8.05 (broad s, 1H), 8.41 (d, 1H) p.p.m.; exact mass calcd. for C₂₆H₂₉CIN₃O₂S (M = N₃) 482.1670, found 482.1673.

### EXAMPLE 20

### N,N-Dimethyl 5-(3-(2-(7-chloroquinolin-2-yl)ethenyl)phenyl)-8-(1H-tetrazol-5-yl)-4-thiaoctanamide

### Step 1 Preparation of methyl 5-(3-cyanophenyl)-4-pentenoate (E:Z mixture)

To 3-carboxypropyl triphenylphosphonium chloride (9.221 g, 24.0 mmoles) in 160 mL THF and 16 mL HMPA, potassium hexamethyldisilazane (0.67M in toluene, 65 mL, 43.5 mmoles) was added dropwise at 0°C. The mixture was stirred 10 minutes at 0°C and 30 minutes at room temperature. Then, at 0°C, 3-cyanobenzaldehyde (2.136 g, 16.3 mmoles) in 25 mL THF was added dropwise and the mixture stirred for 1.5 hours at room temperature. Addition of 25% NH₄0Ac and 2N HCI, extraction with ether, drying, evaporation and flash chromatography of the residue on silica with EtOAc:hexane:AcOH 25:75:1 and 30:70:1 afforded the carboxylic acid. The acid was dissolved in EtOAc:ether and, at 0° C, an excess of diazomethane in ether was added. After 20 hours at 5°C, the solution was washed with 5% NaHC0₃, brine and dried over sodium sulfate. Filtration and evaporation of solvents afforded the title compound as an oil.

¹H NMR (CDCI₃) 8 2.42-2.68 (m, 4H), 3.70 (s, 3H), 5.69-5.82 and 6.23-6.50 (m, 2H, E:Z mixture), 7.35-7.65 (m, 4H) p.p.m.

### Step 2 Preparation of methyl 5-(3-cyanophenyl)-8-(N,N-dimethylcarbamyl-6-thiaoctanoate

At 0°C, AlCl₃ (3.10 g, 5 equiv.) was added to a solution of the cyanostyrene (step 1, 1.005 g, 4.67 mmoles) and N,N-dimethyl 3-mercaptopropanamide (815 mg, 1.3 equiv.) in 50 mL CH₂CI₂. The mixture was stirred 6 hours at room temperature and hydrolyzed with 25% NH₄0Ac and EtOAc. Filtration through celite, extraction and EtOAc, drying, evaporation and flash chromatography of the residue on silica with EtOAc:hexane 50:50, 70:30, 80:20 and 90:10 afforded the title compound.

¹H NMR (CDCI₃) δ1.45-1.97 (m, 4H), 2.30 (t, 2H), 2.40-2.72 (m, 4H), 2.84 (s, 6H), 3.66 (s, 3H), 3.83 (t, 1H), 7.43 (dd, 1H), 7.52-7.62 (m, 2H), 7.66 (broad s, 1H) p.p.m.

### Step 3 Preparation of methyl 8-(N,N-dimethylcarbamyl)-5-(3-formylphenyl)-6-thiaoctanoate

HCI gas was bubbled into a suspension of SnCI2 (5.843 g, 30.8 mmoles) in 30 mL ether until obtention of 2 liquid phases (~15 minutes). Then, the nitrile (step 2, 1.255 g, 3.60 mmoles) dissolved in 5 mL of hot toluene was added. HCI was bubbled through the mixture for another 15 min and the mixture was stirred 3 hours. At 0°C, H₂0 was added and the mixture stirred another hour at room temperature. Addition of 25% NH₄0Ac, filtration through celite, extraction with EtOAc, drying, evaporation and flash chromatography of the residue on silica with acetone:toluene 20:80 afforded the title compound.

¹H NMR (CDCI₃) δ1.48-1.79 (m, 2H), 1.79-2.03 (m, 2H), 2.31 (t, 2H), 2.45 (m, 2H), 2.62 (m, 2H), 2.91 (s, 6H), 3.65 (s, 3H), 3.88 (dd, H),7.51 (dd, H),7.63 (d, 1 H), 7.77 (d, 1 H), 7.86 (broad s, 1 H), 10.02 (s, 1 H) p.p.m.

### Step 4 Preparation of methyl

### 5-(3-(2-(7-chloroquinolin-2-yl)ethenyl)phenyl)-8-(N,N-dimefhylcarbamyl)-6-thiaoctanoate

To (7-chloroquinolin-2-yl) methyltriphenylphosphonium bromide from preparation 2 (2.189 g, 4.22 mmoles) in 18 mL THF at -78° C, n-BuLi (1.6M in hexane, 2.3 mL, 3.68 mmoles) was added dropwise and the dark red solution stirred 1.25 hours at this temperature. The aldehyde (step 3, 0.927 g, 2.64 mmoles) in 7 mL THF was added dropwise and the mixture stirred at -78°C for 30 minutes and at room temperature for 1 hour. The reaction was quenched with 25% NH₄0Ac, extracted with EtOAc, dried and evaporated. The residue was purified by flash chromatography on silica with acetone:toluene 20:80 to afford the title compound.

¹H NMR (CDCI₃) 8 1.52-1.80 (m, 2H), 1.8402.02 (m, 2H), 2.32 (t, 2H), 2.42 (m, 2H), 2.55-2.78 (m, 2H), 2.87 (s, 3H), 2.91 (s, 3H), 3.66 (s, 3H), 3.84 (dd, 1H), 7.26-7.55 (m, 5H), 7.58-7.76 (m, 4H), 8.07 (broad s, 1H), 8.12 (d, 1H) p.p.m.

### Step 5

The ester function of the product of step 4 was converted to the tetrazole in four steps. First, the ester was hydrolyzed to the acid using the procedure of Example 15, step 4, but the reaction time was lengthened to 1 hour. Second, the acid function was converted to the amide using the procedure of Example 11, step 2, but replacing methyl glycinate by an excess of ammonia. Third, the nitrile was formed using the procedure of Example 14, step 1. Finally, the title tetrazole was obtained using the procedure of Example 7, step 5.

¹H NMR (CDCI₃) δ 1.84-1.99 (m, 4H), 2.58 (m, 2H), 2.70 (m, 2H), 3.00 (s, 3H), 3.04 (s, 3H), 3.08 (t, 2H), 3.97 (dd, 1H), 7.22-7.36 (m, 2H), 7.41-7.52 (m, 3H), 7.56 (broad s, 1H), 7.64 (d, 1H), 7.69 (s, 1H), 7.73 (d, 1H), 8.07 (broad s, 1H), 8.12 (d, 1H) p.p.m.; ms 520 (M·+), 477 (M - N₃H), 377 (M -CsH₁₁N₄0), 345 (M - C₅H₁₁N₄OS), 292, 177.

### EXAMPLE 21

### Methyl 4-(3-(7-chloroquinolin-2-ylmethoxy)phenyl)-6-dimethylcarbamyl-5-oxa-hexanoate

### Step 1 3-(Tetrahydropyran-2-yloxy)benzaldehyde

A suspension of 3-hydroxybenzaldehyde (24.4 g, 0.2 mol) in a mixture of methylene chloride (200 mL) and 3,4-dihydro-2H-pyran (27.4 mL, 0.3 mol) was treated with pyridinium p-toluenesulphonate (0.5 g), and the mixture was stirred at room temperature overnight. The resulting dark solution was washed in succession with water, with saturated sodium carbonate solution, and then it was dried over anhydrous potassium carbonate with the addition of charcoal to remove most of the colour. Evaporation of the filtered solution yielded an oil which was purified by column chromatography on Merck neutral alumina eluted with 1:3 ethyl acetate/hexane to afford the product as an oil; nmr (250 MHz, CDC1₃, TMS) 8 10.0 (1 H, s, CHO), 7.57 (1 H, m, ArH), 7.48 (2H, m, ArH), 7.32 (1H, m, ArH), 5.51 (1H, m, O-CH-O), 3.92 and 3.63 (2H, 2m, CH₂-O), 2.2-1.6 (6H, br m, 3xCH₂).

### Step 2 1-(3-(Tetrahydropyran-2-yloxy)phenyl)pent-4-enol

A Grignard reagent was made from 4-bromobut-1-ene (16.88 g, 0.125 mol) and magnesium (3.0 g, 0.125 mol) in dry ether (150 mL), and to this solution, cooled in an ice-bath, was added a solution of the compound from Step 1 (20.6 g, 0.1 mol) in ether (150 mL) dropwise and with stirring. The reaction mixture was stirred for 30 min. in the ice-bath, and then at room temperature for 2 hours. The mixture was cooled again in the ice-bath before the addition of ice-cold 4M ammonium chloride solution. After a further wash with saturated sodium chloride solution, the ether solution was dried over potassium carbonate. The crude product isolated by evaporation of the solvent was purified by column chromatography on Merck silica gel (500 g) eluted with 1:5 ethyl acetate/hexane to give the title compound as an oil; nmr (250 MHz, CDCI₃, TMS) 8 7.26 (1 H, m, ArH), 7.04 (1 H, m, ArH), 6.95 (2H, m, ArH), 5.86 (1H, m, -CH=), 5.43 (1H, m, O-CH-O), 5.00 (2H, m, =CH₂), 4.65 (1 H, m, CH-OH), 3.97 and 3.65 (2H, 2m, CH₂-O), 2.2-1.6 (10H, br m, 5xCH₂).

### Step 3 4-(-(Tetrahydropyran-2-yloxy)phenyl)-3-oxaoct-7-enoic acid

A mixture of the compound from Step 2 (5.24 g, 20 mmol), bromoacetic acid (3.48 g, 25 mmol) and dry tetrahydrofuran (100 mL) was cooled to -78°C under argon, and 0.6M potassium hexamethyl disilazide (83 mL, 50 mmol) was added dropwise with stirring. The pale yellow suspension was stirred at -78°C for 10 min., and then at room temperature for 2 hours. The reaction mixture was diluted with ether, and washed with 2M ammonium chloride solution containing 6N hydrochloric acid (15 mL). The solution was washed three times with sodium chloride solution, and evaporated to ca. 30 mL. This solution was loaded onto a column of Merck silica gel (150 g) which was eluted initially with 1:3 ether/toluene to remove less polar materials, and then with 1:10:100:100 formic acid/t-butanol/ethyl acetate/toluene to obtain the title produce as an oil which used as such for the following step.

### Step 4 N,N-Dimethyl-3-oxa-4-(3-(tetrahydropyran-2-yloxy)phenyl)-oct-7-enamide

A solution of the acid from Step 3 (3.21 g, 10.0 mmol) in methylene chloride (65 mL) was cooled to -15°C under argon, and triethylamine (1.74 mL, 12.5 mmol) was added, followed by isobutyl chloroformate (1.63 mL, 12.5 mmol) added dropwise. The mixture was stirred for 10 min. at -10°C, and then cooled to -15°C again before the addition of 2M dimethylamine in toluene (10 mL). The reaction mixture was stirred at -15°C for 15 min. and then allowed to warm to room temperature. After 30 min., the mixture was washed twice with water, and then with N hydrochloric acid. The organic layer was separated, dried over magnesium sulphate, and evaporated. Column chromatography (Merck silica gel eluted with 1:10:100:200 formic acid/t-butanol/ethyl acetate/toluene) of the crude product afforded the title compound as an oil; nmr (250 MHz, CDCI₃, TMS) 8 7.27 (1H, m, ArH), 6.95 (3H, m, ArH), 5.80 (1H, m, -CH=), 5.41 (1H, m, O-CH-O), 4.95 (2H, m, =CH₂), 4.35 (1H, m, CH-O), 4.07 and 3.88 (2H, 2d, CH₂-CO), 3.9 and 3.6 (2H, m, CH₂-O), 2.92 and 2.91 (6H, 2s, (CH₃)₂N), 2.2-1.5 (10H, br m, 5xCH₂).

Anal. calcd. for C₂₀H₂₉NO₆: C 63.31, H 7.70, N 3.69; found: C 62.94, H 7.95, N 3.51.

### Step 5 Methyl 6-dimethylcarbamyl-5-oxa-4-(3-(tetrahydropyran-2-yloxy)-phenyl)hexanoate

A mixture of the compound from Step 4 (868 mg, 2.5 mmol), potassium carbonate (280 mg), water (30 mL), acetonitrile (20 mL), and carbon tetrachloride (20 mL) was stirred rapidly under argon, and ruthenium dioxide (20 mg) and potassium periodate (2.2 g) were added. After stirring the reaction mixture for 2 hours, more potassium periodate (1.1 g) was added. An hour later, methylene chloride and water were added, and the aqueous phase was made basic with potassium carbonate. The organic phase was separated and washed with water. The combined aqueous extract was acidified with 6N hydrochloric acid, and the acidic product was isolated by extraction with methylene chloride, and esterified immediately with ethereal diazomethane to afford the crude ester, 857 mg, (900/o).

The combined crude product from two such oxidations was purified by flash chromatography (115 g of Merck silica gel eluted with 1:10:100:200 formic acid/t-butanol/ethyl acetate/toluene) to provide the pure title compound as an oil; nmr (250 MHz, CDCI₃, TMS) δ8 7.65 (1 H, m, ArH), 6.95 (3H, m, ArH), 5.40 (1H, m, O-CH-O), 4.37 (1 H, m, CH-O), 4.08 (1 H, d, O-CH-CO), 3.91 (1 H, dd, O-CH-CO), 3.9 and 3.7 (2H, 2m, CH₂-0), 3.65 (3H, s, CH₃-O), 2.92 and 2.91 (6H, 2s, (CH₃)₂N), 2.48 (2H, dd, CH₂-CO), 2.3-1.6 (8H, br m, 4xCH₂).

### Step 6 Methyl 6-dimethylcarbamyl-4-(3-hydroxyphenyl)-5-oxahexanoate

A mixture of the tetrahydropyranyl ether from Step 5 (866 mg, 2.29 mmol), pyridinium p-toluenesulphonate (30 mg), and methanol (17 mL) was heated under reflux for 1 hour, and then evaporated to dryness. The residue was partitioned between methylene chloride and water acidified with N/2 hydrochloric acid (2 mL). The crude product from the organic extract was submitted to column chromatography (30 g of Merck silica gel eluted with 1:10:100:200 formic acid/t-butanol/ethyl acetate/toluene) to afford the title compound as an oil; nmr (250 MHz, CDCl₃, TMS) δ 7.18 (1H, m, ArH), 6.8 (3H, m, ArH), 4.37 (1H, q, CH-O), 4.08 and 3.93 (2H, 2d, CH₂-CO), 3.63 (3H, s, CH₃-O), 2.92 (6H, 2s, (CH₃)₂N), 2.46 and 2.44 (2H, 2d, CH₂-CO), 2.1 (2H, br m, CH₂). Anal. Calcd. for C₁₅H₂₁NO₅: C 61.00, H 7.17, N 4.74; found: C 61.12, H 7.12, N 4.59.

### Step 7 Methyl 4-(3-(7-chloroquinolin-2-ylmethoxy)phenyl)-6-dimethylcarbamyl-5-oxahexanoate

A mixture of the compound from Step 6 (595 mg, 2.01 mmol), 2-bromomethyl-7-chloroquinoline (516 mg, 2.01 mmol), potassium carbonate (552 mg, 4.0 mmol), and acetone (12 mL) was stirred under reflux for 2 hours. The reaction mixture was filtered and evaporated, and the residue was purified by column chromatography (25 g of Merck silica gel eluted with 1:10:100:200 formic acid/t-butanol/ethyl acetate/toluene) to afford the title compound as an oil; nmr (250 MHz, CDCI₃, TMS) δ 8.18 (1 H, d, ArH), 8.08 (3H, d, ArH), 7.76 (1 H, d, ArH), 7.68 (1 H, d, ArH), 7.51 (1 H, dd, ArH), 7.25 (1 H, m, ArH), 7.12 (1 H, m, ArH), 6.93 (2H, m, ArH), 5.36 (2H, s, CH₂-O), 4.42 (1H, m, CH-O), 4.05 and 3.85 (2H, 2d, OCH₂-CO), 3.64 (3H, s, CH₃-0), 2.91 and 2.88 (6H, 2s, (CH₃)₂N), 2.475 and 2.445 (2H, 2d, CH₂-CO), 2.1 (2H, br m, CH₂).

### EXAMPLE 22

### 6-N,N-Dimethylcarbamyl-4-(3-(7-chloroquinolin-2-ylmethoxy)phenyl)-5-oxahexanoic acid

A solution of the title compound from Example 1 (633 mg, 1.305 mmol) in 1,2-dimethoxyethane (6.5 mL) was treated with 2N lithium hydroxide (1.3 mL), and the reaction mixture was stirred overnight at room temperature. The solution was neutralized with formic acid (120 p.L, ca. 2.6 mmol) before evaporation onto silica gel (3.5 g). The solid was placed on top of a column of Merck silica gel (30 g), which was eluted with 1:10:100:200 formic acid/t-butanol/ethyl acetate/toluene to remove unhydrolysed ester, and then with 1:10:100:100 formic acid/t-butanol/ethyl acetate/toluene to isolate the product. The solid from the eluate was recrystallized from acetonitrile to provide the title compound, mp 134-135°C; nmr (250 Mhz, CDCl₃, TMS) 8 8.19 (1H, d, ArH), 8.085 (1H, d, ArH), 7.78 (1H, d, ArH), 7.69 (1H, d, ArH), 7.51 (1H, dd, ArH), 7.28 (1H, m, ArH), 6.95 (3H, br m, ArH), 5.37 (2H, s, CH₂0), 4.41 (1H, dd, cH-O), 4.16 and 3.87 (2H, 2d, CH₂-CO), 2.97 and 2.80 (6H, 2s, (CH₃)₂N), 2.8 and 2.65 (2H, 2m, CH₂COO), 2.0 (2H, br m, CH₂).

Anal. Calcd. for C₂₄H₂sCIN₂0s: C 63.09, H 5.52, Cl 7.76, N 6.13; found: C 62.93, H, 5.55, Cl 7.92, N 6.14.

### EXAMPLE 23

### 8-N,N-Dimethylcarbamyl-6-(3-(7-chloroquinolin-2-ylmethoxy)phenyl)-7-oxaoctanoic acid

### Step 1 Methyl 6-hydroxy-6-(3-(tetrahydropyran-2-yloxy)phenyl}hexanoate

A mixture of the benzaldehyde from Example 1, Step 1 (4.12 g, 20 mmol), methyl 5-bromopentanoate (4.29 g, 22 mmol), finely divided magnesium (600 mg, 2.5 mmol), and dry ether (80 mL) is cooled to -15°C and subjected to ultra-sound until examination by thin layer chromatography suggests that the reaction is not progressing further. The mixture is filtered, evaporated onto silica gel (40 g), and the solid is placed on a column of Merck silica gel (600 g). Elution with 1:3 ethyl acetate/hexane affords the title compound as an oil.

### Step 2 Methyl 7-oxa-6-(3-(tetrahydropyran-2-yloxy)phenyl)dec-9-enoate

To a stirred mixture of the compound from Step 1 (3.22 g, 10 mmol), allyl bromide (1.51 g, 12.5 mmol), and dry tetrahydrofuran (25 mL) cooled to -40°C is added 0.6M potassium hexamethyldisilazide in toluene (18.3 mL) dropwise via a syringe. The reaction mixture is stirred at -40° C for 30 minutes, and then it is allowed to warm slowly to room temperature. Ether is added and the organic solution is washed with water, then with saturated sodium chloride solution, and dried over anhydrous magnesium sulphate. The crude product isolated by evaporation of the solvent is purified by column chromatography (Merck silica gel eluted with 1:5 ethyl acetate/hexane) to afford the title compound as an oil.

### Step 3 8-Methoxycarbonyl-3-oxa-4-(3-(tetrahydropyran-2-yloxy)phenyl)-octanoic acid

Substituting the compound of Step 2 for the compound of Example 1, Step 4 in the procedure of Example 1, Step 5 provides the title compound.

Step 4 Methyl 8-dimethylcarbamyl-7-oxa-6-(3-(tetrahydropyran-2-yloxy)phenyl)octanoate

Substituting the compound of Step 3 for the compound of Example 1, Step 3 in the procedure of Example 1, Step 4 provides the title compound.

### Step 5 Methyl 8-dimethylcarbamyl-7-oxa-6-(3-hydroxyphenyl)octanoate

Substituting the compound of Step 4 for the compound of Example 1, Step 5 in the procedure of Example 1, Step 6 affords the title compound.

### Step 6 Methyl 6-(3-(7-chloroquinolin-2-ylmethoxy)phenyl)-8-dimethylcarbamyl-7-oxaoctanoate

Substituting the compound of Step 5 for the compound of Example 1, Step 6 in the procedure of Example 1, Step 7 provides the title compound.

### Step 7 8-N,N-Dimethylcarbamyl-6-(3-(7-chloroquinolin-2ylmethoxy)phenyl)-7-oxaoctanoic acid

Substituting the compound of Step 6 for the compound of Example 1, Step 7 in the procedure of Example 1, Step 8 affords the title compound.

### EXAMPLE 24

### 8-N,N-dimethylcarbamyl-6-(3-(7-chloroquinolin-2ylmethoxy)phenyl)-7-oxaoctanoic acid (alternate synthesis)

Using the procedures on of Example 21, steps 2 to 7 and Example 22, but using the Grignard reagent made from 6-bromohex-1-ene instead of 4-bromobut-1-ene, the title compound is prepared.

## Claims

1. A compound of the formula: 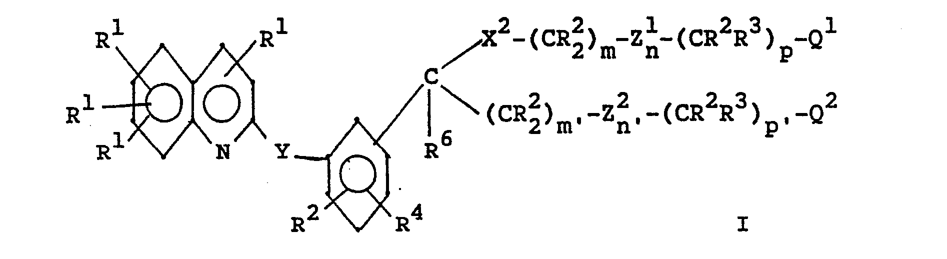 wherein:
R¹ is H, halogen, C₁-C₈ alkyl, C₂-Ca alkenyl, C₂-C₈ alkynyl, -CF₃, -OR², -SR², -S(O)R², -S(O)₂R², -NR²R², -CHO, -COOR², -(C=O)R2, -C(OH)R2R², -CN, -N0₂, substituted or unsubstituted phenyl, substituted or unsubstituted benzyl, or substituted or unsubstituted phenethyl;
R² is H, C₁-Ca alkyl, C₂-C₈ alkenyl, C₂-C₈ alkynyl, -CF₃, substituted or unsubstituted phenyl, substituted or unsubstituted benzyl, or substituted or unsubstituted phenethyl;
R³ is H, halogen, -N0₂, -CN, -OR², -SR², NR²R², or C₁-C₈ alkyl;
CR²R³ may be the radical of a naturally occurring amino acid;
R⁴ is H, halogen, -N0₂, -CN, -OR², -SR², NR²R², C₁-C₈ alkyl, or -(C=O)R²;
R5 is 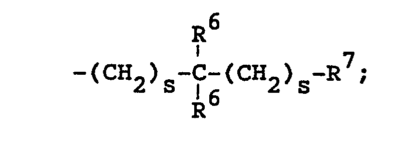
R⁶ is H or C₁-C₄ alkyl;
R⁷ is A) a monocyclic or bicyclic heterocyclic radical containing from 3 to 12 nuclear carbon atoms and 1 or 2 nuclear heteroatoms selected from N, S or 0 and with each ring in the heterocyclic radical being formed of 5 or 6 atoms, or
B) the radical W-R^{8;}
R⁸ contains up to 21 carbon atoms and is (1) a hydrocarbon radical or (2) an acyl radical of an organic
acyclic or monocyclic carboxylic acid containing not more than 1 heteroatom in the ring;
R⁹ is-OR¹⁰, -SR¹⁰, or NR¹⁰R¹⁰;
R¹⁰ is H, Ci-C₆ alkyl, -(C=O)R¹¹, unsubstituted phenyl, unsubstituted benzyl, or two R¹⁰ groups joined to the same N may form a ring of 5 or 6 members containing up to two heteratoms chosen from O,S or N; R¹¹ is H, c₁-C₈ alkyl, C₂-C₈ alkenyl, C₂-C₈ alkynyl, -CF₃, or unsubstituted phenyl, benzyl, or phenethyl; R¹² is R² or halogen;
m and m'are independently 0-8;
n and n' are independently 0 or 1;
p and p' are independently 0-8;
m+n+p is 1-10;
m'+n'+p' is 0-10;
s is 0-3;
Q¹ and Q² are independently -COOR², tetrazole, -COOR⁵, -CONHS(O)₂R¹¹, -CN, -CONR¹⁰R¹⁰, -CHO,
-CH₂0H, -COCH₂0H, -NHS(O)₂R¹¹; or if Q¹ or Q² is COOH and R³ is -OH, -SH, or -NHR² then Q¹ or Q² and R³ and the carbons through which they are attached may form a heterocyclic ring with loss of water; W is O, S, or NH;
X¹ is 0, S, -S(O)-, -S(O)₂-, -NR², or -CR²R²-;
X² is CR²R², O, S, S(O), or S(O)2;
Y is -CR²=CR²-, -C=C-, -CR²R²-X¹-, -X¹-CR²R²-, -CR²R²-X¹-CR²R²-, 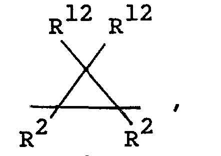 C=O, 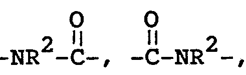 O, S, or -_{NR}²_{;}
Z¹ and Z² are independently -CONR²⁻;
and the pharmaceutically acceptable salts thereof.

2. A compound of Claim 1 of the Formula la: 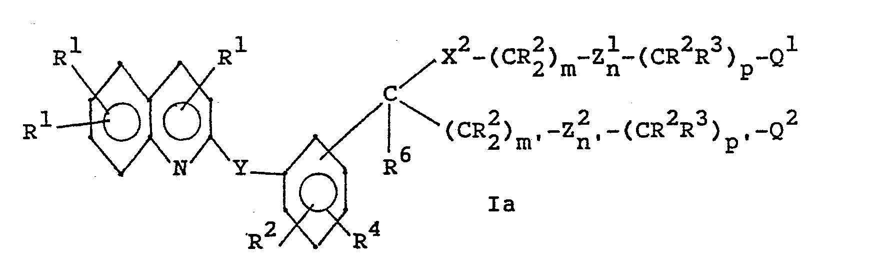 wherein:
Y is -CR²=CR²-, -C=C-, 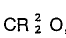 , or 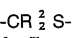 ;
the other substituents are as defined for Formula I;
and the pharmaceutically acceptable salts thereof.

3. A compound of Claim 1 of the Formula Ib: 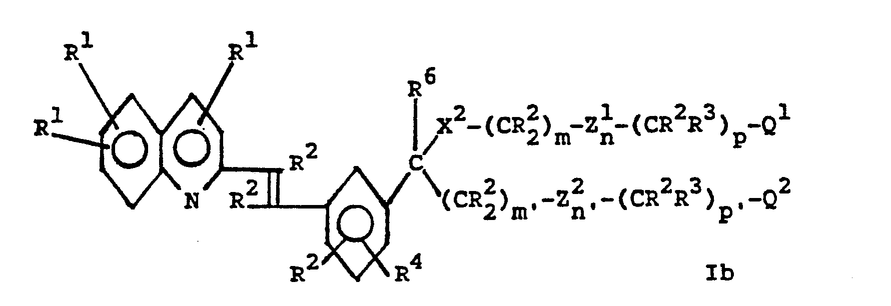 wherein:
R¹ is H, halogen, C₁-C₃alkyl,-CF₃, or SCF₃;
R² is H, C₁-C₃ alkyl, C₂-C₃ alkenyl, or -CF₃;
the other substituents are as defined for Formula I; and the pharmaceutically acceptable salts thereof.

4. A compound of Claim 1 of the Formula Ic: 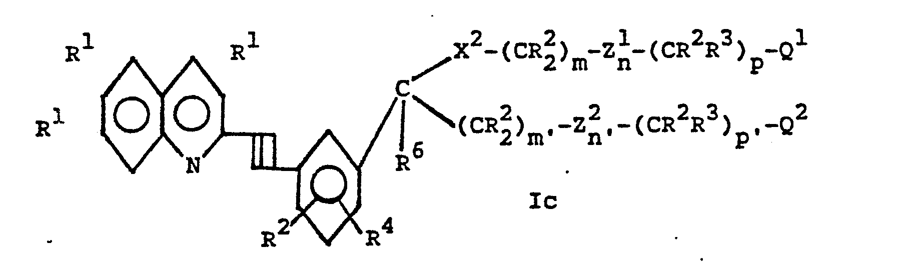 wherein:
R¹ is H, halogen, C₁-C₃ alkyl, -CF₃, or SCF₃;
R² is H, C₁-C₃ alkyl, C₂-C₃ alkenyl, or-CF3;
the other substituents are as defined for Formula I; and the pharmaceutically acceptable salts thereof.

5. A compound of Claim 1 of the Formula Id: 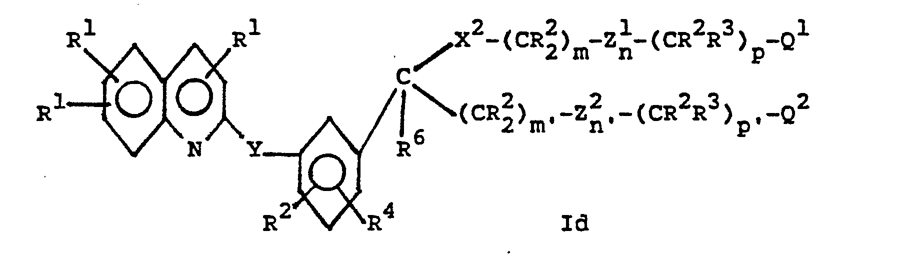 wherein:
R¹ is H, halogen, C₁-C₃ alkyl, CF₃ or SCF₃;
R² is H, C₁-C₃alkyl, C₂-C₃alkenyl, or CF₃;
Y is 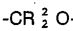 - or 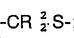 ;
the other substituents are as defined for Formula I; and the pharmaceutically acceptable salts thereof.

6. A compound of Claim 1 of Formula le which is:

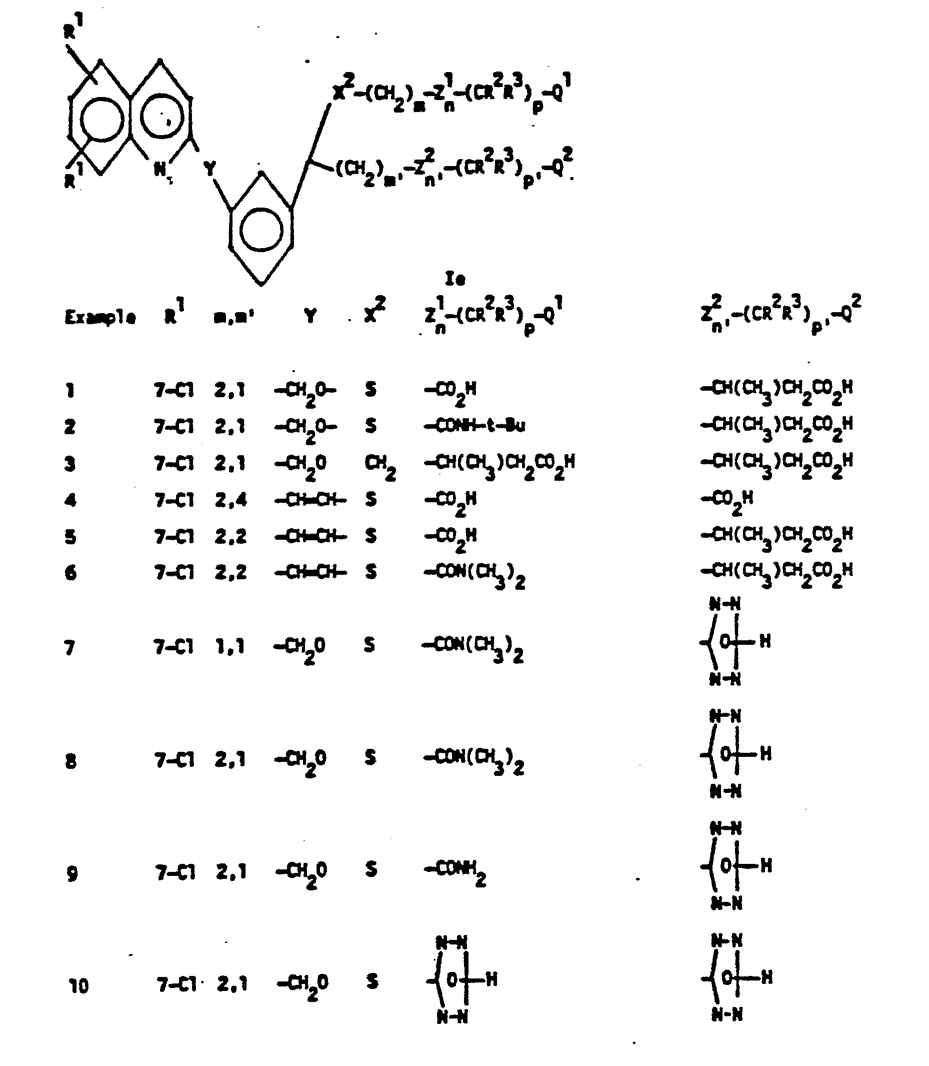

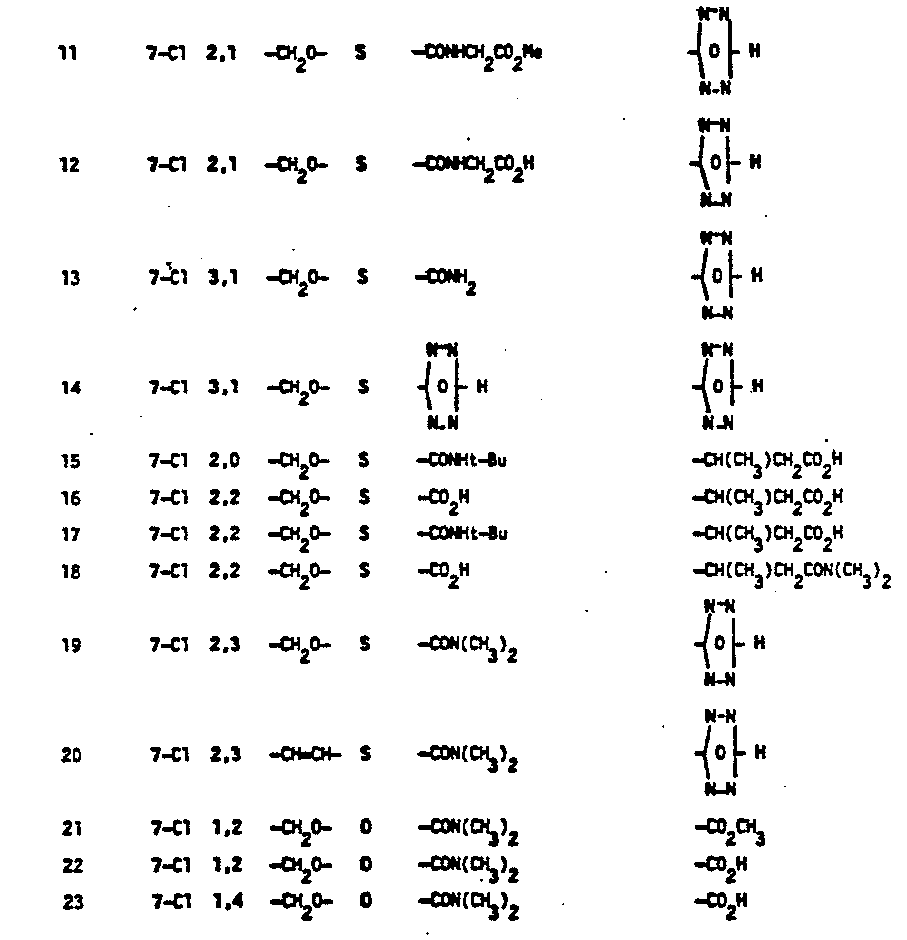

7. A pharmaceutical composition useful in antagonizing leukotriene action in mammals comprising an amount of a compound of Claim 1 effective as a leukotriene antagonist and a pharmaceutically acceptable carrier.

8. The pharmaceutical composition of Claim 7 additionally comprising an effective amount of a second active ingredient selected from the group consisting of non-steroidal anti-inflammatory drugs; peripheral analgesic agents; cyclooxygenase inhibitors; leukotriene antagonists; leukotriene inhibitors; H₂-receptor antagonists; antihistaminic agents; prostaglandin antagonists; thromboxane antagonists; and serotonin antagonists.

9. The use of a compound of Claim 1 for the preparation of a medicament useful for preventing the synthesis, the action, or the release of SRS-A or leukotriene D₄ in mammals.

10. The use of a compound of Claim 1 for the preparation of a medicament useful for treating inflammatory diseases of the eye in mammals.
